(19) Europäisches Patentamt
European Patent Office
Office européen des brevets

(11) EP 2 982 687 A1

(12) **EUROPEAN PATENT APPLICATION**

(43) Date of publication:
**10.02.2016 Bulletin 2016/06**

(21) Application number: **15183054.4**

(22) Date of filing: **31.07.2009**

(51) Int Cl.:
***C07K 14/62*** *(2006.01)* ***C12N 15/17*** *(2006.01)*
***A61K 38/28*** *(2006.01)*

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **31.07.2008 US 85212 P**

(62) Document number(s) of the earlier application(s) in accordance with Art. 76 EPC:
**09803678.3 / 2 318 432**

(71) Applicant: **Case Western Reserve University**
**Cleveland, OH 44106 (US)**

(72) Inventor: **WEISS, Michael**
**Moreland Hills, OH 44022 (US)**

(74) Representative: **Pearson, Samuel John**
**Abel & Imray**
**20 Red Lion Street**
**London WC1R 4PQ (GB)**

Remarks:
This application was filed on 28-08-2015 as a divisional application to the application mentioned under INID code 62.

(54) **HALOGEN-STABILIZED INSULIN**

(57) An insulin analogue comprising a B-chain polypeptide incorporating a halogenated phenylalanine at position B24, B25 or B26. The analogue may be of a mammalian insulin such as human insulin. A nucleic acid encoding such an insulin analogue. Some halogenated insulin analogues retain significant activity. Medical uses of the insulin analogues for treating a patient comprises administering a physiologically effective amount of the insulin analogue or a physiologically acceptable salt thereof to a patient. Halogen substitution-based stabilisation of insulin may enhance the treatment of diabetes mellitus in regions of the developing world lacking refrigeration. The preferred analogue is an insulin analogue comprising a B-chain polypeptide incorporating a pentafluoro-phenylalanine (5F-Phe) at position B24.

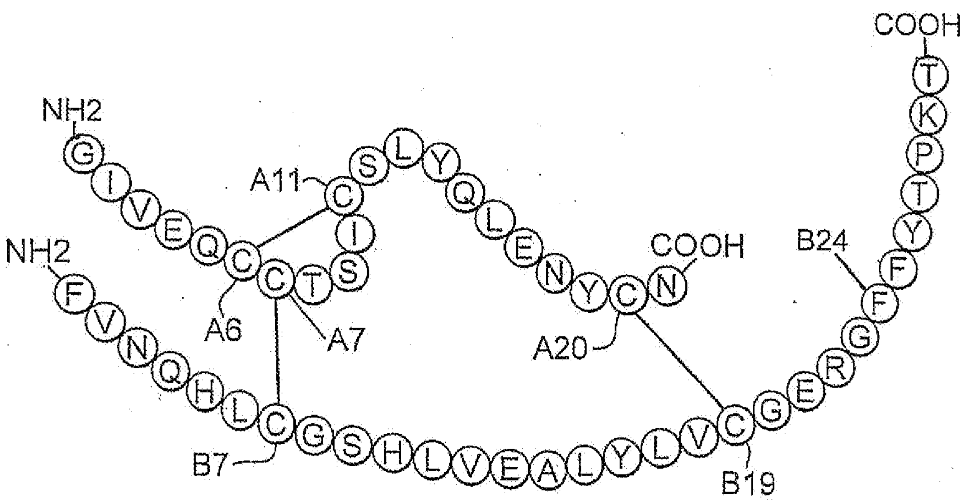


**FIG. 1C**

(PRIOR ART)



Printed by Jouve, 75001 PARIS (FR)

SEQUENCE LISTING

<110> Case Western Reserve University

<120> HALOGEN-STABILIZED INSULIN

<130> 200512.00075

<150> US 61/085,212
<151> 2008-07-31

<160> 26

<170> PatentIn version 3.5

<210> 1
<211> 86
<212> PRT
<213> Homo sapiens

<400> 1

```
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85
```

<210> 2
<211> 21
<212> PRT
<213> Homo sapiens

<400> 2

```
Gly Ile Val Glu Gln Cys Cys Thr Ser Ile Cys Ser Leu Tyr Gln Leu
1               5                   10                  15

Glu Asn Tyr Cys Asn
            20
```

<210> 3
<211> 30

```
<212>  PRT
<213>  Homo sapiens

<400>  3

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr
            20                  25                  30


<210>  4
<211>  30
<212>  PRT
<213>  Homo sapiens


<220>
<221>  MISC_FEATURE
<222>  (24)..(26)
<223>  One Phe is halogenated

<220>
<221>  MISC_FEATURE
<222>  (26)..(26)
<223>  The residue at this position may be Phe or Tyr

<400>  4

Phe Val Asn Gln His Leu Cys Gly Ser His Leu Val Glu Ala Leu Tyr
1               5                   10                  15

Leu Val Cys Gly Glu Arg Gly Phe Phe Phe Thr Pro Lys Thr
            20                  25                  30


<210>  5
<211>  30
<212>  PRT
<213>  Homo sapiens


<220>
<221>  MISC_FEATURE
<222>  (24)..(26)
<223>  One Phe is halogenated

<220>
<221>  MISC_FEATURE
<222>  (26)..(26)
<223>  The residue at this postion may be Phe or Tyr

<400>  5

Phe Val Asn Gln His Leu Cys Gly Ser His Leu Val Glu Ala Leu Tyr
1               5                   10                  15

Leu Val Cys Gly Glu Arg Gly Phe Phe Phe Thr Lys Pro Thr
            20                  25                  30
```

<210>  6
<211>  30
<212>  PRT
<213>  Homo sapiens

<220>
<221>  MISC_FEATURE
<222>  (24)..(26)
<223>  One Phe is halogenated

<220>
<221>  MISC_FEATURE
<222>  (26)..(26)
<223>  The residue at this postion may be Phe or Tyr

<400>  6

```
Phe Val Asn Gln His Leu Cys Gly Ser His Leu Val Glu Ala Leu Tyr
1               5                   10                  15

Leu Val Cys Gly Glu Arg Gly Phe Phe Phe Thr Asp Lys Thr
            20                  25                  30
```

<210>  7
<211>  86
<212>  PRT
<213>  Homo sapiens

<220>
<221>  MISC_FEATURE
<222>  (1)..(1)
<223>  The residue at this postion may be His or Phe

<220>
<221>  MISC_FEATURE
<222>  (24)..(26)
<223>  One Phe is halogenated

<220>
<221>  MISC_FEATURE
<222>  (26)..(26)
<223>  The residue at this postion may be Tyr or Phe

<220>
<221>  MISC_FEATURE
<222>  (28)..(28)
<223>  The residue at this postion may be Pro, Lys or Asp

<220>
<221>  MISC_FEATURE
<222>  (29)..(29)
<223>  The residue at this postion may be Lys or Pro

<220>
<221>  VARIANT
<222>  (31)..(65)
<223>  Any 0 to 35 residues can be present, or a break in the amino acid

chain may be present

<220>
<221> MISC_FEATURE
<222> (31)..(65)
<223> Residue at any positions 31-65 may be any amino acid residue; no relative information regarding relative frequency of alternative residues is implied.

<220>
<221> MISC_FEATURE
<222> (69)..(69)
<223> The residue at this postion may be His or Glu

<220>
<221> MISC_FEATURE
<222> (73)..(73)
<223> The residue at this postion may be His or Thr

<400> 7

```
His Val Asn Gln His Leu Cys Gly Ser His Leu Val Glu Ala Leu Tyr
1               5                   10                  15

Leu Val Cys Gly Glu Arg Gly Phe Phe Phe Thr Asp Lys Thr Ala Ala
            20                  25                  30

Ala Ala Ala Ala Ala Ala Ala Ala Ala Ala Ala Ala Ala Ala Ala Ala
        35                  40                  45

Ala Ala Ala Ala Ala Ala Ala Ala Ala Ala Ala Ala Ala Ala Ala Ala
    50                  55                  60

Ala Gly Ile Val Glu Gln Cys Cys His Ser Ile Cys Ser Leu Tyr Gln
65                  70                  75                  80

Leu Glu Asn Tyr Cys Asn
                85
```

<210> 8
<211> 30
<212> PRT
<213> Homo sapiens

<220>
<221> MISC_FEATURE
<222> (24)..(26)
<223> One Phe is halogenated

<220>
<221> MISC_FEATURE
<222> (26)..(26)
<223> The residue at this postion may be Tyr or Phe

<400> 8

Phe Val Asn Gln His Leu Cys Gly Ser Asp Leu Val Glu Ala Leu Tyr
1 5 10 15

Leu Val Cys Gly Glu Arg Gly Phe Phe Phe Thr Lys Pro Thr
20 25 30

<210> 9
<211> 8
<212> PRT
<213> Homo sapiens

<220>
<221> MISC_FEATURE
<222> (2)..(3)
<223> One Phe is halogenated

<400> 9

Gly Phe Phe Tyr Thr Lys Pro Thr
1 5

<210> 10
<211> 8
<212> PRT
<213> Homo sapiens

<220>
<221> MISC_FEATURE
<222> (2)..(3)
<223> One Phe is halogenated

<400> 10

Gly Phe Phe Tyr Thr Pro Lys Thr
1 5

<210> 11
<211> 8
<212> PRT
<213> Artificial

<220>
<223> Synthetic Construct

<400> 11

Asp Tyr Lys Asp Asp Asp Asp Lys
1 5

<210> 12
<211> 90
<212> DNA
<213> Homo sapiens

<400> 12
tttgtgaacc aacacctgtg cggctcacac ctggtggaag ctctctacct agtgtgcggg 60

gaacgaggct agttctacac acccaagacc 90

<210> 13
<211> 90
<212> DNA
<213> Artificial

<220>
<223> Synthetic Construct

<400> 13
tttgttaacc aacatttgtg tggttctcat ttggttgaag ctttgtactt ggtttgtggt 60

gaaagaggtt agttttacac tccaaagact 90

<210> 14
<211> 261
<212> DNA
<213> Homo sapiens

<400> 14
tttgtgaacc aacacctgtg cggctcacac ctggtggaag ctctctacct agtgtgcggg 60

gaacgaggct agttctacac acccaagacc cgccgggagg cagaggacct gcaggtgggg 120

caggtggagc tgggcggcgg ccctggtgca ggcagcctgc agcccttggc cctggagggg 180

tccctgcaga agcgtggcat tgtggaacaa tgctgtacca gcatctgctc cctctaccag 240

ctggagaact actgcaacta g 261

<210> 15
<211> 261
<212> DNA
<213> Artificial

<220>
<223> Synthetic Construct

<400> 15
tttgttaacc aacatttgtg tggttctcat ttggttgaag ctttgtactt ggtttgtggt 60

gaaagaggtt agttttacac tccaaagact agaagagaag ctgaagattt gcaagttggt 120

caagttgaat tgggtggtgg tccaggtgct ggttctttgc aaccattggc tttggaaggt 180

tctttgcaaa agagaggtat tgttgaacaa tgttgtactt ctatttgttc tttgtaccaa 240

ttggaaaact actgtaacta a 261

<210> 16
<211> 22
<212> PRT
<213> Homo sapiens

<400> 16

Phe Val Asn Gln His Leu Cys Gly Ser Asp Leu Val Glu Ala Leu Tyr

1 5 10 15

Leu Val Cys Gly Glu Arg
20

<210> 17
<211> 22
<212> PRT
<213> Homo sapiens

<400> 17

Phe Val Asn Gln His Leu Cys Gly Ser His Leu Val Glu Ala Leu Tyr
1 5 10 15

Leu Val Cys Gly Glu Arg
20

<210> 18
<211> 8
<212> PRT
<213> Homo sapiens

<220>
<221> MISC_FEATURE
<222> (4)..(4)
<223> Phe is halogenated

<400> 18

Gly Phe Phe Phe Thr Lys Pro Thr
1 5

<210> 19
<211> 90
<212> DNA
<213> Homo sapiens

<400> 19
tttgtgaacc aacacctgtg cggctcacac ctggtggaag ctctctacct agtgtgcggg 60

gaacgaggct tctagtacac acccaagacc 90

<210> 20
<211> 90
<212> DNA
<213> Artificial

<220>
<223> Synthetic Construct

<400> 20
tttgttaacc aacatttgtg tggttctcat ttggttgaag ctttgtactt ggtttgtggt 60

gaaagaggtt tttagtacac tccaaagact 90

<210> 21
<211> 261
<212> DNA
<213> Homo sapiens

<400> 21
tttgtgaacc aacacctgtg cggctcacac ctggtggaag ctctctacct agtgtgcggg 60

gaacgaggct tctagtacac acccaagacc cgccgggagg cagaggacct gcaggtgggg 120

caggtggagc tgggcggcgg ccctggtgca ggcagcctgc agcccttggc cctggagggg 180

tccctgcaga agcgtggcat tgtggaacaa tgctgtacca gcatctgctc cctctaccag 240

ctggagaact actgcaacta g 261

<210> 22
<211> 261
<212> DNA
<213> Artificial

<220>
<223> Synthetic Construct

<400> 22
tttgttaacc aacatttgtg tggttctcat ttggttgaag ctttgtactt ggtttgtggt 60

gaaagaggtt tttagtacac tccaaagact agaagagaag ctgaagattt gcaagttggt 120

caagttgaat tgggtggtgg tccaggtgct ggttctttgc aaccattggc tttggaaggt 180

tctttgcaaa agagaggtat tgttgaacaa tgttgtactt ctatttgttc tttgtaccaa 240

ttggaaaact actgtaacta a 261

<210> 23
<211> 90
<212> DNA
<213> Homo sapiens

<400> 23
tttgtgaacc aacacctgtg cggctcacac ctggtggaag ctctctacct agtgtgcggg 60

gaacgaggct tcttctagac acccaagacc 90

<210> 24
<211> 90
<212> DNA
<213> Artificial

<220>
<223> Synthetic Construct

<400> 24
tttgttaacc aacatttgtg tggttctcat ttggttgaag ctttgtactt ggtttgtggt 60

gaaagaggtt ttttttagac tccaaagact 90

<210> 25
<211> 261
<212> DNA
<213> Homo sapiens

<400> 25
tttgtgaacc aacacctgtg cggctcacac ctggtggaag ctctctacct agtgtgcggg 60

gaacgaggct tcttctagac acccaagacc cgccgggagg cagaggacct gcaggtgggg 120

caggtggagc tgggcggcgg ccctggtgca ggcagcctgc agcccttggc cctggagggg 180

tccctgcaga agcgtggcat tgtggaacaa tgctgtacca gcatctgctc cctctaccag 240

ctggagaact actgcaacta g 261

<210> 26
<211> 261
<212> DNA
<213> Artificial

<220>
<223> Synthetic Construct

<400> 26
tttgttaacc aacatttgtg tggttctcat ttggttgaag ctttgtactt ggtttgtggt 60

gaaagaggtt ttttttagac tccaaagact agaagagaag ctgaagattt gcaagttggt 120

caagttgaat tgggtggtgg tccaggtgct ggttctttgc aaccattggc tttggaaggt 180

tctttgcaaa agagaggtat tgttgaacaa tgttgtactt ctatttgttc tttgtaccaa 240

ttggaaaact actgtaacta a 261

## Description

CROSS-REFERENCE TO RELATED APPLICATION

This application claims the benefit of U.S. Provisional Application No. 61/085,212 filed on July 31, 2008.

STATEMENT REGARDING FEDERALLY SPONSORED RESEARCH OR DEVELOPMENT

This invention was made with government support under cooperative agreements awarded by the National Institutes of Health under grant numbers DK40949 and DK074176. The U.S. government may have certain rights to the invention.

BACKGROUND OF THE INVENTION

This invention relates to polypeptide analogues that are resistant to thermal degradation. More particularly, this invention relates to thermally stabilized insulin analogues. Even more particularly, this invention relates to insulin analogues that are chemically and thermally stabilized by the incorporation of the element fluorine, chlorine, or bromine into an amino acid of the insulin analogue. These elements are classified as *halogens* and are distinguished from the ordinary constituents of proteins by their atomic radius, electronegativity, stereoelectronic distribution of partial charges, and transmitted effects on the stereoelectronic properties of neighboring atoms in a molecule.

The engineering of ultra-stable proteins, including therapeutic agents and vaccines, may have broad societal benefits in regions of the developing world where electricity and refrigeration are not consistently available. An example of a therapeutic protein susceptible to thermal degradation is provided by insulin. The challenge posed by its chemical and physical degradation is deepened by the pending epidemic of diabetes mellitus in Africa and Asia. Because chemical degradation rates of insulin analogues correlate inversely with their relative stabilities, the design of ultra-stable formulations may enhance the safety and efficacy of insulin replacement therapy in such challenged regions.

The utility of fluorous substitutions in small organic molecules is known in medicinal chemistry. Fluorinated functional groups are critical to the efficacy of such widely prescribed small molecules as *atorvastatin* (Liptor™), an inhibitor of cholesterol biosynthesis, and *fluoxetine hydrochloride* (Prozac™), a selective serotonin reuptake inhibitor used in the treatment of depression and other affective disorders. Although the atomic radius of fluorine is similar to that of hydrogen, its large inductive effects modify the stereo-electronic properties of these drugs, in turn enhancing their biological activities. Similar considerations of physical organic chemistry pertain to the incorporation of larger halogen atoms, such as chlorine and bromine. The small molecule *montelukast sodium* (Singulair™) is a leukotriene inhibitor whose pharmaceutical properties are enhanced by covalent incorporation of a chlorine atom.

Attention has previously focused on the use of multiply fluorinated aliphatic side chains (such as trifluoro-$\gamma$-$CF_3$-Val, trifluoro-$\delta$-$CF_3$-Val, trifluoro- $\delta$-$CF_3$-Ile, hexafluoro-$\gamma_{1,2}$-$CF_3$- Val, and hexafluoro-$\delta_{1,2}$-$CF_3$-Leu) to maximize the gain in hydrophobicity associated with this modification. An example is provided by the stabilization of a model $\alpha$-helical motif, the homodimeric coiled coil. Its interfacial aliphatic chains were simultaneously substituted by trifluoro-analogues, creating a fluorous core whose stability is enhanced by 0.3 - 2.0 kcal/mole. The degree of stabilization per fluorine atom is < 0.1 kcal/mole. More marked stabilization per fluorine atom has been achieved in an unrelated $\alpha$-helical domain by substitution of a single internal Phe by pentafluoro-Phe ($F_5$-Phe)[1] ($\Delta\Delta G_u$ 0.6 kcal/mole per five fluorine atoms). Stabilization occurs only at one specific position in the protein, suggesting that its mechanism requires a particular spatial environment. The structure of a related $F_5$-Phe-modified domain is identical to that of the unmodified domain. Structural stabilization however, is only a portion of the requirements for a biologically active polypeptide. At least a significant portion of the activity must also be maintained.

An extensive literature describes the use of fluorine labels in proteins as $^{19}$F-NMR probes. Whereas such labels are widely regarded as non-perturbing, applications of fluorinated amino acids in protein engineering seek to exploit their altered physicochemical properties. Studies of a model $\alpha$-helical fold (the villin headpiece subdomain) containing single $F_5$-Phe substitutions have demonstrated that whether and to what extent such a modification may affect protein stability depends in detail on structural environment. Indeed, the apparent stability of this model fold was enhanced by an $F_5$-Phe substitution only at one of the seven sites tested in the core. It should be noted however, that this stabilizing effect was only demonstrated in a nonstandard polypeptide analogue containing a sulfur atom *in the main chain* near the site of fluorination; in our hands the self-same $F_5$-Phe substitution in villin headpiece subdomain with native polypeptide main chain has no effect on its stability. Thus, to our knowledge, *bona fide* data demonstrating enhancement of protein stability due to halogenation of an aromatic residue in an otherwise native protein has not previously been reported. These observations suggest that a generally hydrophobic environment does not in itself assure that the modification will be stabilizing. Because protein interiors are often stabilized by aromatic-aromatic interactions with a specific distance- and angular dependence, a subset of stabilizing $F_5$-Phe substitutions may adopt particularly favorable perfluroaryl/aryl geometries. Such interactions arise from the asymmetric distribution of partial charges in these aromatic systems. Modulation of the chemical, physical, and biological properties of proteins by the site-specific incorporation of chlorine or bromine atoms into modified amino acids are less well characterized in the scientific literature than are the above effects of incorporation of fluorine atoms.

Aromatic side chains may engage in a variety of weakly polar interactions, involving not only neighboring aromatic rings but also other sources of positive- or negative electrostatic potential. Examples include main-chain carbonyl- and amide groups in peptide bonds.

Administration of insulin has long been established as a treatment for diabetes mellitus. Insulin is a small globular protein that plays a central role in metabolism in vertebrates. Insulin contains two chains, an A chain, containing 21 residues and a B chain containing 30 residues. The hormone is stored in the pancreatic β-cell as a $Zn^{2+}$-stabilized hexamer, but functions as a $Zn^{2+}$-free monomer in the bloodstream. Insulin is the product of a single-chain precursor, proinsulin, in which a connecting region (35 residues) links the C-terminal residue of B chain (residue B30) to the N-terminal residue of the A chain (Fig. 1A). Although the structure of proinsulin has not been determined, a variety of evidence indicates that it consists of an insulin-like core and disordered connecting peptide (Fig. 1B). Formation of three specific disulfide bridges (A6-A11, A7-B7, and A20-B19; Figs. 1A and 1B) is thought to be coupled to oxidative folding of proinsulin in the rough endoplasmic reticulum (ER). Proinsulin assembles to form soluble $Zn^{2+}$-coordinated hexamers shortly after export from ER to the Golgi apparatus. Endoproteolytic digestion and conversion to insulin occurs in immature secretory granules followed by morphological condensation. Crystalline arrays of zinc insulin hexamers within mature storage granules have been visualized by electron microscopy (EM).

Amino-acid substitutions in insulin have been investigated for effects on thermodynamic stability and biological activity. No consistent relationship has been observed between stability and activity. Whereas some substitutions that enhance thermodynamic stability also enhance binding to the insulin receptor, other substitutions that enhance stability impede such binding. The effects of substitution of $Thr^{A8}$ by several other amino acids has been investigated in wild-type human insulin and in the context of an engineered insulin monomer containing three unrelated substitutions in the B-chain ($His^{B10}$→Asp, $Pro^{B28}$→Lys, and $Lys^{B29}$→Pro) have been reported. Examples are also known in the art of substitutions that accelerate or delay the time course of absorption. Such substitutions (such as $Asp^{B28}$ in Novalog® and [$Lys^{B28}$, $Pro^{B29}$] in Humalog®) can be and often are associated with more rapid fibrillation and poorer physical stability. Indeed, a series of ten analogues of human insulin have been tested for susceptibility to fibrillation, including $Asp^{B28}$-insulin and $Asp^{B10}$-insulin. All ten were found to be more susceptible to fibrillation at pH 7.4 and 37° C than is human insulin. The ten substitutions were located at diverse sites in the insulin molecule and are likely to be associated with a wide variation of changes in classical thermodynamic stability. Although a range of effects has been observed, no correlation exists between activity and thermodynamic stability.

Insulin is a small globular protein that is highly amenable to chemical synthesis and semi-synthesis, which facilitates the incorporation of nonstandard side chains. Insulin contains three phenylalanine residues (positions B1, B24, and B25) and a structurally similar tyrosine at position B26. Conserved among vertebrate insulins and insulin-like growth factors, the aromatic ring of $Phe^{B24}$ packs against (but not within) the hydrophobic core to stabilize the super-secondary structure of the B-chain. $Phe^{B24}$ lies at the classical receptor-binding surface and has been proposed to direct a change in conformation on receptor binding. $Phe^{B25}$ projects from the surface of the insulin monomer whereas $Tyr^{B26}$ packs near aliphatic side chains ($Ile^{A2}$, $Val^{A3}$, and $Val^{B12}$) at one edge of the core. The B24-related conformational change is proposed to enable $Phe^{B25}$ and $Tyr^{B26}$ to contact distinct domains of the insulin receptor.

The present theory of protein fibrillation posits that the mechanism of fibrillation proceeds via a partially folded intermediate state, which in turn aggregates to form an amyloidogenic nucleus. In this theory, it is possible that amino-acid substitutions that stabilize the native state may or may not stabilize the partially folded intermediate state and may or may not increase (or decrease) the free-energy barrier between the native state and the intermediate state. Therefore, the current theory indicates that the tendency of a given amino-acid substitution in the insulin molecule to increase or decrease the risk of fibrillation is highly unpredictable.

Fibrillation, which is a serious concern in the manufacture, storage and use of insulin and insulin analogues for diabetes treatment, is enhanced with higher temperature, lower pH, agitation, or the presence of urea, guanidine, ethanol co-solvent, or hydrophobic surfaces. Current US drug regulations demand that insulin be discarded if fibrillation occurs at a level of one percent or more. Because fibrillation is enhanced at higher temperatures, diabetic individuals optimally must keep insulin refrigerated prior to use. Fibrillation of insulin or an insulin analogue can be a particular concern for diabetic patients utilizing an external insulin pump, in which small amounts of insulin or insulin analogue are injected into the patient's body at regular intervals. In such a usage, the insulin or insulin analogue is not kept refrigerated within the pump apparatus and fibrillation of insulin can result in blockage of the catheter used to inject insulin or insulin analogue into the body, potentially resulting in unpredictable blood glucose level fluctuations or even dangerous hyperglycemia. At least one recent report has indicated that lispro insulin (an analogue in which residues B28 and B29 are interchanged relative to their positions in wild-type human insulin; trade name Humalog®) may be particularly susceptible to fibrillation and resulting obstruction of insulin pump catheters.

Insulin fibrillation is an even greater concern in implantable insulin pumps, where the insulin would be contained within the implant for 1-3 months at high concentration and at physiological temperature (i.e. 37° C), rather than at ambient temperature as with an external pump. Additionally, the agitation caused by normal movement would also tend to accelerate fibrillation of insulin. In spite of the increased potential for insulin fibrillation, implantable insulin pumps are still the subject of research efforts, due to the potential advantages of such systems. These advantages include intraperitoneal delivery of insulin to the portal circulatory system, which mimics normal physiological delivery of insulin more closely than subcutaneous injection, which provides insulin to the patient via the systemic circulatory system. Intraperitoneal delivery provides more rapid and consistent absorption of insulin compared to subcutaneous injection, which can provide variable absorption and degradation from one injection site to another. Administration of insulin via an implantable pump also potentially provides increased patient convenience. Whereas efforts to prevent fibrillation, such as by addition of a surfactant to the reservoir, have provided some improvement, these improvements have heretofore been considered insufficient to allow reliable usage of an implanted insulin pump in diabetic patients outside of strictly monitored clinical trials.

As noted above, the developing world faces a challenge regarding the safe storage, delivery, and use of drugs and vaccines. This challenge complicates the use of temperature-sensitive insulin formulations in regions of Africa and Asia lacking consistent access to electricity and refrigeration, a challenge likely to be deepened by the pending epidemic of diabetes in the developing world. Insulin exhibits an increase in degradation rate of 10-fold or more for each 10° C increment in temperature above 25° C, and guidelines call for storage at temperatures < 30° C and preferably with refrigeration. At higher temperatures insulin undergoes both chemical degradation (changes in covalent structure such as formation of iso-aspartic acid, rearrangement of disulfide bridges, and formation of covalent polymers) and physical degradation (non-native aggregation and fibrillation.

Amino-acid substitutions have been described in insulin that stabilize the protein but augment its binding to the insulin receptor (IR) and its cross-binding to the homologous receptor for insulin-like growth factors (IGFR) in such a way as to confer a risk of carcinogenesis. An example known in the art is provided by the substitution of $His^{B10}$ by aspartic acid. Although $Asp^{B10}$-insulin exhibits favorable pharmaceutical properties with respect to stability and pharmacokinetics, its enhanced receptor-binding properties were associated with tumorigenesis in Sprague-Dawley rats. Although there are many potential substitutions in the A- or B chains that can be introduced into $Asp^{B10}$-insulin or related analogues to reduce its binding to IR and IGFR to levels similar to that of human insulin, such substitutions generally impair the stability of insulin (or insulin analogues) and increase its susceptibility to chemical and physical degradation. It would be desirable to discover a method of modification of insulin and of insulin analogues that enabled "tuning" of receptor-binding affinities while at the same time enhancing stability and resistance to fibrillation. Such applications would require a set of stabilizing modifications that reduce binding to IR and IGFR to varying extent so as to offset the potential carcinogenicity of analogues that are super-active in their receptor-binding properties.

Therefore, there is a need for insulin analogues that are stabilized by the incorporation of a non-standard amino acid containing a halogen atom as a nonstandard functional group while maintaining at least a portion of the biological activity of the analogue.

## SUMMARY OF THE INVENTION

It is, therefore, an aspect of the present invention to provide an insulin analogue that provides greater stability by halogen substitution in an amino acid, where the analogue then maintains at least a portion of biological activity of the corresponding non-halogenated insulin or insulin analogue.

In general, the present invention provides an insulin analogue comprising a B-chain polypeptide which incorporates a halogenated phenylalanine at position B24, B25, or B26. In one embodiment, the halogenated phenylalanine is located at position B24. In another embodiment, the halogenated phenylalanine is *ortho*-monofluoro-phenylalanine, *ortho*-monobromo-phenylalanine or *ortho*-monochloro-phenylalanine. In another embodiment, the insulin analogue is a mammalian insulin analogue, such as an analogue of human insulin. In one particular set of embodiments, the B-chain polypeptide comprises an amino acid sequence selected from the group consisting of SEQ. ID. NOS. 4-8 and polypeptides having three or fewer additional amino acid substitutions thereof.

Also provided is a nucleic acid encoding an insulin analogue comprising a B-chain polypeptide that incorporates a halogenated phenylalanine at position B24, B25, or B26. In one example, the halogenated phenylalanine is encoded by a stop codon, such as the nucleic acid sequence TAG. An expression vector may comprise such a nucleic acid and a host cell may contain such an expression vector.

The invention also provides a method of treating a patient. The method comprises administering a physiologically effective amount of an insulin analogue or a physiologically acceptable salt thereof to the patient, wherein the insulin analogue or a physiologically acceptable salt thereof contains a B-chain polypeptide incorporating a halogenated phenylalanine at position B24, B25, or B26. In one embodiment, the halogenated phenylalanine in the insulin analogue administered to a patient is located at position B24. In another embodiment, the halogenated phenylalanine is *or-*

*tho*-monofluoro-phenylalanine, *ortho*-monobromo-phenylalanine or *ortho*-monochloro-phenylalanine. In still another embodiment, the insulin analogue is a mammalian insulin analogue, such as an analogue of human insulin. In one particular set of embodiments, the B-chain polypeptide comprises an amino acid sequence selected from the group consisting of SEQ. ID. NOS. 4-8 and polypeptides having three or fewer additional amino-acid substitutions thereof.

BRIEF DESCRIPTION OF THE SEVERAL VIEWS OF THE DRAWINGS

FIG. 1A is a schematic representation of the sequence of human proinsulin including the A- and B-chains and the connecting region shown with flanking dibasic cleavage sites (filled circles) and C-peptide (open circles).

FIG. 1B is a structural model of proinsulin, consisting of an insulin-like moiety and a disordered connecting peptide (dashed line).

FIG. 1C is a schematic representation of the sequence of human insulin indicating the position of residue B24 in the B-chain.

FIG. 1D is a ribbon model of an insulin monomer showing aromatic residue of $Phe^{B24}$ in relation to the three disulfide bridges. The adjoining side chains of $Leu^{B15}$ (arrow) and $Phe^{B24}$ are shown. The A- and B-chain chains are otherwise shown in light and dark gray, respectively, and the sulfur atoms of cysteines as circles.

FIG. IE is a space-filling model of insulin showing the $Phe^{B24}$ side chain within a pocket at the edge of the hydrophobic core.

FIG. 2A is a representation of pentafluoro-phenylalanine ($F_5$-Phe).

FIG. 2B is a representation of *ortho*-monofluoro-phenylalanine (2F-Phe).

FIG. 2C is a representation of *meta*-monofluoro-phenylalanine (3F-Phe).

FIG. 2D is a representation of *para*-monofluoro-phenylalanine (4F-Phe).

FIG. 3 is a set of four circular dichroism (CD) spectra for the far-UV wavelengths. Panel A: DKP-insulin (solid black line) and 2F-$Phe^{B24}$-DKP-insulin(□); Panel B: DKP-insulin (solid black line) and 3F-$Phe^{B24}$-DKP-insulin(▲); Panel C: DKP-insulin (solid black line) and 4F-$Phe^{B24}$-DKP-insulin (▼); Panel D: DKP-insulin (solid black line) and $F_5$-$Phe^{B24}$-DKP-insulin (●).

FIG. 4 is a set of four graphs for CD-detected guanidine denaturation studies. Panel A: DKP-insulin (solid black line) and 2F-$Phe^{B24}$-DKP-insulin (□); Panel B: DKP-insulin (solid black line) and 3F-$Phe^{B24}$-DKP-insulin (A); Panel C: DKP-insulin (solid black line) and 4F-$Phe^{B24}$-DKP-insulin (▼); Panel D: DKP-insulin (solid black line) and $F_5$-$Phe^{B24}$-DKP-insulin (●).

FIG. 5 is a graph showing the results of receptor-binding studies of insulin analogues. Relative activities are determined by competitive binding assay in which receptor-bound $^{125}$I-labeled human insulin is displaced by increasing concentrations of DKP-insulin (■) or its analogues: pentafluoro-$Phe^{B24}$ (▲), 2F-$Phe^{B24}$ (▼), 3F-$Phe^{B24}$ (♦), and 4F-$Phe^{B24}$ (●).

FIG. 6 is a graph showing the results of receptor-binding studies of insulin analogues. Relative activities are determined by competitive binding assay in which receptor-bound $^{125}$I-labeled human insulin is displaced by increasing concentrations of KP-insulin (■) or its analogues: 2Br-$Phe^{B24}$-KP-insulin (A), 2Cl-$Phe^{B24}$-KP-insulin (▼), 2F-$Phe^{B24}$-KP-insulin (♦), and 4F-$Phe^{B24}$-KP-insulin (●). Assay employed the B-isoform of the insulin receptor and $^{125}$I-$Tyr^{A14}$-human insulin as tracer.

FIG. 7A is a graph for CD-detected guanidine denaturation studies of human insulin (solid line), KP-insulin (dashed line; "parent"), KP-insulin analogues containing monofluorous substitution of 2F-$Phe^{B24}$-KP-insulin (▲), 3F-$Phe^{B24}$-KP-insulin (■), and 4F-$Phe^{B24}$-KP-insulin (○).

FIG. 7B is a graph for CD-detected guanidine denaturation studies of human insulin (solid line), KP-insulin (dashed line; "parent"), 2-Cl-$Phe^{B24}$-KP-insulin (▼) and 2-Br-$Phe^{B24}$-KP-insulin (Δ).

FIG. 8A is a $^1$H-NMR spectra for DKP-insulin analogues comparing fluoro-substitutions at positions 2, 3, and 4 of $Phe^{B24}$ in relation to DKP-insulin, recorded at 700 MHz at 32° C and pD 7.0.

FIG. 8B is a NMR spectra for KP-insulin analogues comparing fluoro-, bromo- and chloro-substitutions at position 2 of PheB24 in relation to KP-insulin, recorded at 700 MHz at 32° C and pD 7.0.

DETAILED DESCRIPTION OF THE INVENTION

The present invention is directed an insulin analogue that provides greater stability by halogen substitution in an amino acid, where the analogue then maintains at least a portion of biological activity of the corresponding non-halogenated insulin or insulin analogue. Particularly, the present invention provides insulin analogues that provides greater stability by substitution of a single halogen in an amino acid, where the analogue then maintains at least a portion of biological activity of the corresponding non-halogenated insulin or insulin analogue. In one example, the present invention provides an insulin analogue that provides greater stability by fluorine, chlorine or bromine substitution in an amino acid, where the analogue then maintains at least a portion of biological activity of the corresponding non-halogenated insulin or insulin analogue. One potential application is to augment the chemical and physical stability of an insulin analogue while retaining a portion of its biological activity; another application is to calibrate the receptor-binding properties of an insulin analogue so not to exceed that of human insulin. To these ends, the present invention provides insulin analogues that contain a halogenated phenylalanine (Phe) residue as a substitution at position B24, B25 or B26. While substitutions of a halogenated phenylalanine at B24 or B25 do not change the underlying amino acid sequence of insulin or an insulin analogue, a halogenated phenylalanine at position B26 of insulin must substitute for tyrosine (Tyr) found in the wild-type sequence of insulin.

The present invention is not limited, however, to human insulin and its analogues. It is also envisioned that these substitutions may also be made in animal insulins such as porcine, bovine, equine, and canine insulins, by way of non-limiting examples.

Furthermore, in view of the similarity between human and animal insulins, and use in the past of animal insulins in human diabetic patients, it is also envisioned that other minor modifications in the sequence of insulin may be introduced, especially those substitutions considered "conservative." For example, additional substitutions of amino acids may be made within groups of amino acids with similar side chains, without departing from the present invention. These include the neutral hydrophobic amino acids: Alanine (Ala or A), Valine (Val or V), Leucine (Leu or L), Isoleucine (Ile or I), Proline (Pro or P), Tryptophan (Tip or W), Phenylalanine (Phe or F) and Methionine (Met or M). Likewise, the neutral polar amino acids may be substituted for each other within their group of Glycine (Gly or G), Seline(Ser or S), Threonine (Thr or T), Tyrosine (Tyr or Y), Cysteine (Cys or C), Glutamine (Glu or Q), and Asparagine (Asn or N). Basic amino acids are considered to include Lysine (Lys or K), Arginine (Arg or R) and Histidine (His or H). Acidic amino acids are Aspartic acid (Asp or D) and Glutamic acid (Glu or E). Unless noted otherwise or wherever obvious from the context, the amino acids noted herein should be considered to be L-amino acids. In one example, the insulin analogue of the present invention contains three or fewer conservative substitutions other than the halogenated-Phe substitutions of the present invention. In another example, the insulin analogue of the present invention contains one or fewer conservative substitutions other than the halogenated-Phe substitutions of the present invention.

As used in this specification and the claims, various amino acids in insulin or an insulin analogue may be noted by the amino acid residue in question, followed by the position of the amino acid, optionally in superscript. The position of the amino acid in question includes the A or B chain of insulin where the substitution is located. Thus, $Phe^{B24}$ denotes a phenylalanine at the twenty fourth amino acid of the B chain of insulin, while $Phe^{B25}$ denotes a phenylalanine at the twenty fifth amino acid of the B chain of insulin, and $Phe^{B26}$ denotes a phenylalanine substitution for tyrosine at the twenty sixth amino acid of the B chain of insulin. A fluorinated amino acid may be indicated with the prefix "F-," a brominated amino acid may be indicated with the prefix "Br-" and a chlorinated amino acid may be indicated with the prefix "Cl-." Therefore, fluorinated phenylalanine may be abbreviated "F-Phe," chlorinated phenylalanine may be abbreviated "Cl-Phe" and brominated phenylalanine may be abbreviated "Br-Phe." In the case of phenylalanine, the position of the halogen substituents or substituents on the phenyl side chain may be further indicated by the number of the carbon to which the halogen is attached. Therefore, *ortho*-monofluoro-phenylalanine (shown in Fig. 2B) is abbreviated "2F-Phe," *meta*-monofluoro-phenylalanine (shown in Fig. 2C) is abbreviated "3F-Phe" and *para*-monofluoro-phenylalanine (shown in Fig. 2D) is abbreviated 4F-Phe. Pentafluoro-phenylalanine (shown in Fig. 2A) is abbreviated as "$F_5$-Phe." Similarly, *ortho*-monobromo-phenylalanine may be abbreviated "2Br-Phe," *ortho*-monochloro-phenylalanine may be abbreviated "2Cl-Phe."

The phenylalanine at B24 is an invariant amino acid in functional insulin and contains an aromatic side chain. The biological importance of $Phe^{B24}$ in insulin is indicated by a clinical mutation ($Ser^{B24}$) causing human diabetes mellitus. As illustrated in Figs. 1D and IE, and while not wishing to be bound by theory, $Phe^{B24}$ is believed to pack at the edge of a hydrophobic core at the classical receptor binding surface. The models are based on a crystallographic protomer (2-Zn molecule 1; Protein Databank identifier 4INS). Lying within the C-terminal β-stand of the B-chain (residues B24-B28), $Phe^{B24}$ adjoins the central α-helix (residues B9-B19). One face and edge of the aromatic ring sit within a shallow pocket defined by $Leu^{B15}$ and $Cys^{B19}$; the other face and edge are exposed to solvent (Fig. IE). This pocket is in part surrounded by main-chain carbonyl and amide groups and so creates a complex and asymmetric electrostatic environment. The effects of the near-symmetric substituent tetrafluoro-phenylalanine ($F_5$-$Phe^{B24}$) (with its enhanced general hydrophobicity and fluoraryl-aryl interactions;) (Fig. 2A) with those of "unbalanced" analogues containing single *ortho*-, *meta*-, or *para*-fluorous substitutions (designated 2F-Phe, 3F-Phe, or 4F-Phe, Figs. 2B-2D, respectively) on insulin stability are provided.

$Phe^{B25}$ is thought to lie at the surface of the insulin monomer and in solution structures projects into solvent with less structural organization than that of $Phe^{B24}$. Its biological importance is likewise indicated by a clinical mutation ($Leu^{B25}$) causing human diabetes. $Tyr^{B26}$, like $Phe^{B24}$, lies at the edge of the hydrophobic core where it packs near nonpolar residues $Ile^{A2}$, $Val^{A3}$, and $Val^{B12}$. Photo-cross-linking studies suggest that the side chains of residues B24, B25, and B26 each contact the insulin receptor. In dimers and hexamers of insulin residues, residues B24-B26 and symmetry-related residues B24'-B26' are believed to participate in an intermolecular anti-parallel β-sheet. Aromatic side chains at these positions are thought to stabilize packing between monomers at this β-sheet interface.

It is envisioned that the substitutions of the present invention may be made in any of a number of existing insulin analogues. For example, the halogenated phenylalanine (X-Phe) substitutions provided herein may be made in insulin analogues such as Lispro insulin, insulin Aspart, other modified insulins or insulin analogues, or within various pharmaceutical formulations, such as regular insulin, NPH insulin, lente insulin or ultralente insulin, in addition to human insulin. Aspart insulin contains an $Asp^{B28}$ substitution and is sold as Novalog® whereas Lispro insulin contains $Lys^{B28}$ and $Pro^{B29}$ substitutions and is known as and sold under the name Humalog®. These analogues are described in US Pat. Nos. 5,149,777 and 5,474,978, the disclosures of which are hereby incorporated by reference herein. Both of these analogues are known as fast-acting insulins.

A halogenated-Phe substitution at B24, B25 and/or B26 may also be introduced into analogues of human insulin that, while not previously clinically used, are still useful experimentally, such as DKP insulin, described more fully below, or miniproinsulin, a proinsulin analogue containing a dipeptide (Ala-Lys) linker between the A chain and B chain portions of insulin in place of the normal 35 amino acid connecting region between the C-terminal residue of the B chain and the N-terminal residue of the A chain. Incorporation of halogenated aromatic residues at positions B24, B25, or B26 in DKP-insulin (or other insulin analogues that contain $Asp^{B10}$ or that exhibit receptor-binding affinities higher than that of human insulin) can reduce their receptor-binding affinities to be similar to or below that of human insulin and so potentially enable clinical use. In this manner the cross-binding of insulin analogues to the mitogenic IGFR may also be reduced.

The amino-acid sequence of human proinsulin is provided, for comparative purposes, as SEQ. ID. NO. 1.

SEQ. ID. NO. 1 (proinsulin)
Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Tyr-Thr-Pro-Lys-Thr-Arg-Arg-Glu-Ala-Glu-Asp-Leu-Gln-Val-Gly-Gln-Val-Glu-Leu-Gly-Gly-Gly-Pro-Gly-Ala-Gly-Ser-Leu-Gln-Pro-Leu-Ala-Leu-Glu-Gly-Ser-Leu-Gln-Lys-Arg-Gly-Ile-Val-Glu-Gln-Cys-Cys-Thr-Ser-Ile-Cys-Ser-Leu-Tyr-Gln-Leu-Glu-Asn-Tyr-Cys-Asn

The amino acid sequence of the A chain of human insulin is provided as SEQ. ID. NO. 2.

SEQ. ID. NO. 2 (A chain)
Gly-Ile-Val-Glu-Gln-Cys-Cys-Thr-Ser-Ile-Cys-Ser-Leu-Tyr-Gln-Leu-Glu-Asn-Tyr-Cys-Asn

The amino acid sequence of the B chain of human insulin is provided as SEQ. ID. NO. 3.

SEQ. ID. NO. 3 (B chain)
Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Tyr-Thr-Pro-Lys-Thr

The amino acid sequence of a B chain of human insulin may be modified with a substitution of a halogenated-Phe at position B24, B25, or B26. An example of such a sequence is provided As SEQ. ID. NO 4, where Xaa may be Tyr or Phe. The halogen used in any of these substitutions may be fluorine, chlorine or bromine, for example.

SEQ. ID. NO. 4
Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Xaa-Thr-Pro-Lys-Thr
[Xaa is Tyr or Phe]

Further combinations of other substitutions are also within the scope of the present invention. It is also envisioned that the substitutions of the present invention may also be combined with substitutions of prior known insulin analogues. For example, the amino acid sequence of an analogue of the B chain of human insulin containing the $Lys^{B28}$ $Pro^{B29}$ substitutions of lispro insulin (Humalog®), in which one of the halogenated Phe substitution may also be introduced, is provided as SEQ. ID. NO. 5. Likewise, the amino acid sequence of an analogue of the B chain of human insulin containing the $Asp^{B28}$ substitution of aspart insulin, in which the $F\text{-}Phe^{B24}$ substitution may also be introduced, is provided as SEQ. ID. NO. 6.

SEQ. ID. NO. 5
Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Xaa-Thr-Lys-Pro-Thr
[Xaa is Tyr or Phe]

SEQ. ID. NO. 6
Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Xaa-Thr-Asp-Lys-Thr
[Xaa is Tyr or Phe]

The F-Phe$^{B24}$ substitution may also be introduced in combination with other insulin analogue substitutions such as analogues of human insulin containing His substitutions at residues A4, A8 and/or B1 as described more fully in co-pending International Application No. PCT/US07/00320 and US Application Ser. No. 12/160,187, the disclosures of which are incorporated by reference herein. For example, the F-Phe$^{B24}$ substitution may be present with [His$^{A4}$, His$^{A8}$], and/or His$^{B1}$ substitutions in an insulin analogue or proinsulin analogue having the amino acid sequence represented by SEQ. ID. NO. 7,

SEQ. ID. NO. 7

R1-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-R2-Thr-R3-R4-Thr- $Xaa_{0-35}$- Gly-Ile-Val-R5-Gln-Cys-Cys-R6-Ser-Ile-Cys-Ser-Leu-Tyr-Gln-Leu-Glu-Asn-Tyr-Cys-Asn;

wherein R1 is His or Phe; wherein R2 is Tyr or Phe, R3 is Pro, Lys, or Asp; wherein R4 is Lys or Pro; wherein R5 is His or Glu; wherein R6 is His or Thr; and wherein $Xaa_{0-35}$ is 0-35 of any amino acid or a break in the amino acid chain; and further wherein at least one substitution selected from the group of the following amino acid substitutions is present:

R1 is His; and
R6 is His; and
R5 and R6 together are His.

A halogenated-Phe substitution at B24, B25, or B26 may also be introduced into a single chain insulin analogue as disclosed in co-pending US Provisional Patent Application No. 60/828,153, the disclosure of which is incorporated by reference herein.

Fluorine substitutions were introduced within an engineered insulin monomer of high activity, designated DKP-insulin, which contains the substitutions Asp$^{B10}$ (D), Lys$^{B28}$ (K) and Pro$^{B29}$ (P). These three substitutions on the surface of the B-chain are believed to impede formation of dimers and hexamers. Use of an engineered monomer circumvents confounding effects of self-assembly on stability assays. The structure of DKP-insulin closely resembles a crystallographic protomer. The sequence of the B-chain polypeptide for DKP insulin is provided as SEQ. ID. NO. 8, where Xaa is Tyr or Phe.

SEQ. ID. NO. 8 (DKP B-Chain Sequence)
Phe-Val-Asn-Gln-His-Leu-Cys-Gly-Ser-Asp-Leu-Val-Glu-Ala-Leu-Tyr-Leu-Val-Cys-Gly-Glu-Arg-Gly-Phe-Phe-Xaa-Thr-Lys-Pro-Thr

Analogues of DKP-insulin were prepared by trypsin-catalyzed semi-synthesis and purified by high-performance liquid chromatography (Mirmira, R.G., and Tager, H.S., 1989. J. Biol. Chem. 264: 6349-6354.) This protocol employs (i) a synthetic octapeptide representing residues (N)-GF*FYTKPT (including modified residue (F*) and "KP" substitutions (underlined); SEQ. ID. NO. 9) and (ii) truncated analogue *des*-octapeptide[B23-B30]-Asp$^{B10}$-insulin (SEQ. ID. NO. 16). Because the octapeptide differs from the wild-type B23-B30 sequence (GFFYT*PK*T; SEQ. ID. NO. 10) by interchange of Pro$^{B28}$ and Lys$^{B29}$ (italics), protection of the lysine ε-amino group is not required during trypsin treatment. In brief, *des*-octapeptide (15 mg) and octapeptide (15 mg) were dissolved in a mixture of dimethylacetamide/1,4-butandiol/0.2 M Tris acetate (pH 8) containing 10 mM calcium acetate and 1 mM ethylene diamine tetra-acetic acid (EDTA) (35:35:30, v/v, 0.4 mL). The final pH was adjusted to 7.0 with 10 μL of N-methylmorpholine. The solution was cooled to 12 °C, and 1.5 mg of TPCK-trypsin was added and incubated for 2 days at 12 °C. An additional 1.5 mg of trypsin was added after 24 hr. The reaction was acidified with 0.1% trifluoroacetic acid and purified by preparative reverse-phase HPLC (C4). Mass spectrometry using matrix-assisted laser desorption/ionization time-of-flight (MALDI-TOF; Applied Biosystems, Foster City, CA) in each case gave expected values (not shown). The general protocol for solid-phase synthesis is as described (Merrifield et al., 1982. Biochemistry 21: 5020-5031). 9-fluoren-9-yl-methoxycarbonyl (F-moc)-protected phenylalanine analogues were purchased from Chem-Impex International (Wood Dale, IL).

Circular dichroism (CD) spectra were obtained at 4° C and 25° C using an Aviv spectropolarimeter (Weiss et al., Biochemistry 39: 15429-15440). Samples contained *ca.* 25 μM DKP-insulin or analogues in 50 mM potassium phosphate (pH 7.4); samples were diluted to 5 μM for guanidine-induced denaturation studies at 25° C. To extract free energies of unfolding, denaturation transitions were fitted by non-linear least squares to a two-state model as described by Sosnick et al., Methods Enzymol. 317: 393-409. In brief, CD data θ(*x*), where x indicates the concentration of denaturant, were fitted by a nonlinear least-squares program according to

$$\theta(x) = \frac{\theta_A + \theta_B e^{(-\Delta G^o_{H_2O} - mx)/RT}}{1 + e^{-(\Delta G^o_{H_2O} - mx)/RT}}$$

where x is the concentration of guanidine and where $\theta_A$ and $\theta_B$ are baseline values in the native and unfolded states. Baselines were approximated by pre- and post-transition lines $\theta_A(x) = \theta_A^{H_2O} + m_A x$ and $\theta_B(x) = \theta_B^{H_2O}$ The *m* values obtained in fitting the variant unfolding transitions are lower than the *m* value obtained in fitting the wild-type unfolding curve. To test whether this difference and apparent change in $\Delta G_u$ result from an inability to measure the CD signal from the fully unfolded state, simulations were performed in which the data were extrapolated to plateau CD values at higher concentrations of guanidine; essentially identical estimates of $\Delta G_u$ and *m* were obtained.

Relative activity is defined as the ratio of analogue to wild-type human insulin required to displace 50 percent of specifically bound $^{125}$I-human insulin. A human placental membrane preparation containing the insulin receptor (IR) was employed as known in the art. Membrane fragments (0.025 mg protein/tube) were incubated with $^{125}$I-labeled insulin (ca. 30,000 cpm) in the presence of selected concentrations of unlabelled analogue for 18 hours at 4° C in a final volume of 0.25 ml of 0.05 M Tris-HCl and 0.25 percent (w/v) bovine serum albumin at pH 8. Subsequent to incubation, mixtures are diluted with 1 ml of ice-cold buffer and centrifuged (10,000g) for 5 min at 4° C. The supernatant is then removed by aspiration, and the membrane pellet counted for radioactivity. Data is corrected for nonspecific binding (amount of radioactivity remaining membrane associated in the presence of 1 μM human insulin. In all assays the percentage of tracer bound in the absence of competing ligand was less than 15% to avoid ligand-depletion artifacts. An additional insulin receptor-binding assay to monitor changes in activity during the course of incubation of the insulin analogue at 37° C was performed using a microtiter plate antibody capture as known in the art. Microtiter strip plates (Nunc Maxisorb) were incubated overnight at 4° C with AU5 IgG (100 μl/well of 40 mg/ml in phosphate-buffered saline). Binding data were analyzed by a two-site sequential model. A corresponding microtiter plate antibody assay using the IGF Type I receptor was employed to assess cross-binding to this homologous receptor.

The far-ultraviolet circular dichroism (CD) spectra of the singly fluorinated analogues are essentially identical to that of the parent analogue (Fig. 3); a slight distortion is observed in the spectrum of the $F_5$-Phe$^{B24}$ analogue (Fig. 3, Panel D). The stabilities and receptor-binding activities of the analogues are provided in Table 1. Modified B24 residues were introduced within the context of DKP-insulin. Activity values shown are based on ratio of hormone-receptor dissociation constants relative to human insulin; the activity of human insulin is thus 1.0 by definition. Standard errors in the activity values were in general less than 25%. Free energies of unfolding ($\Delta G_u$) at 25° C were estimated based on a two-state model as extrapolated to zero denaturant concentration. Lag time indicates time (in days) required for initiation of protein fibrillation on gentle agitation at 37° C in zinc-free phosphate-buffered saline (pH 7.4).

Table 1

Activities and Stabilities of Insulin Analogues

| analog | activity | $\Delta G_u$ (kcal/mole) | $C_{mid}$ (M) | *m* (kcal mol$^{-1}$ M$^{-1}$) | lag time |
|---|---|---|---|---|---|
| DKP-insulin | 2.42 | 4.0 ± 0.1 | 5.6 ± 0.1 | 0.70 ± 0.01 | 12.4 ± 2.5 |
| $F_5$-Phe$^{B24}$-DKP | 0.013 | 4.8 ± 0.1 | 5.8 ± 0.1 | 0.84 ± 0.02 | 17.7 ± 2.1 |
| 2F-Phe$^{B24}$-DKP | 0.37 | 4.9 ± 0.1 | 5.8 ± 0.1 | 0.85 ± 0.02 | 16.0 ± 0.1 |
| 3F-Phe$^{B24}$-DKP | 1.02 | 3.8 ± 0.1 | 5.5 ± 0.2 | 0.70 ± 0.02 | 17.7 ± 3.7 |

(continued)

Activities and Stabilities of Insulin Analogues

| analog | activity | $\Delta G_u$ (kcal/mole) | $C_{mid}$ (M) | $m$ (kcal $mol^{-1}$ $M^{-1}$) | lag time |
|---|---|---|---|---|---|
| 4F-$Phe^{B24}$-DKP | 0.43 | 3.9 ± 0.1 | 5.6 ± 0.2 | 0.70 ± 0.02 | 11.0 ± 1.6 |

Substitution of $Phe^{B24}$ by $F_5$-Phe augments the stability of DKP-insulin by 0.8 ± 0.1 kcal/mole at 25° C (Fig. 4, Panel D). Despite such a favorable effect on stability, the activity of the analogue is negligible (< 1% receptor-binding affinity relative to the parent analog; Table 1 and Fig. 5). This impairment is more marked than that ordinarily observed on standard single-amino-acid substitution. By contrast, the singly substituted analogues each retain significant activity: *ortho* (37% relative to human insulin), *meta* (100%), and *para* (43%). Such activities are each above the threshold of 10% (corresponding to a hormone-receptor dissociation constant of < 1 nM) generally predictive of therapeutic efficacy. Further, whereas the 3F-$Phe^{B24}$ and 4F-$Phe^{B24}$ insulin analogues are no more stable (and possibly slightly less stable) than the parent analogue (Fig. 4 and Table 1), 2F-$Phe^{B24}$-DKP-insulin is at least as stable as the $F_5$-Phe analogue ($\Delta\Delta G_u$ 0.9 ± 0.1 kcal/mole). The physical stabilities of the analogues were evaluated in triplicate during incubation in 60 μM phosphate-buffered saline (PBS) at pH 7.4 at 37° C under gentle agitation. The samples were observed for 20 days or until signs of precipitation or frosting of the glass vial were observed. $F_5$-, 2F- and 3F-$Phe^{B24}$-DKP-insulin analogues (but not 4F- $Phe^{B24}$-DKP-insulin) each exhibit an increase in lag time (relative to the parent analogue) prior to onset of protein fibrillation (Table 1). Substitution of a single fluorine atom is thus able to augment the stability of a monomeric insulin analogue with retention of reduced but clinically significant activity; the modification can also extend the lag time prior to protein fibrillation on gentle agitation at pH 7.4 and 37° C.

Whereas the $F_5$- $Phe^{B24}$ substitution is highly stabilizing, this insulin analogue is essentially without biological activity. Remarkably, the regiospecific modification 2F-$Phe^{B24}$ is equally stabilizing while retaining substantial (although reduced) affinity for the insulin receptor.

Fluorine substitutions were also introduced, essentially as described above, with the exception of *ortho*-, *meta*-, *para-,* and penta-fluorinated phenylalanine being introduced at positions B24, B25 and B26 in lispro insulin (that is, an insulin analogue also containing the substitutions $Lys^{B28}$, $Pro^{B29}$ (sold under the name Humalog®)). Analogues containing *ortho*-, *meta*-, *para-,* and penta-fluorinated phenylalanine substitutions at position B24 are designated 2F-$Phe^{B24}$-KP-insulin, 3F-$Phe^{B24}$-KP-insulin, 4F-$Phe^{B24}$-KP-insulin, F5-$Phe^{B24}$-KP-insulin, respectively. Analogues containing *ortho*-, *meta*-, *para-,* and penta-fluorinated phenylalanine substitutions at position B25 are designated 2F-$Phe^{B25}$-KP-insulin, 3F-$Phe^{B25}$-KP-insulin, 4F-$Phe^{B25}$-KP-insulin, F5-$Phe^{B25}$-KP-insulin, respectively. Analogues containing *ortho*-, *meta*-, *para-,* and penta-fluorinated phenylalanine substitutions at position B26 (substituting for tyrosine) are designated 2F-$Phe^{B26}$-KP-insulin, 3F-$Phe^{B26}$-KP-insulin, 4F-$Phe^{B26}$-KP-insulin, $F_5$-$Phe^{B26}$-KP-insulin, respectively. Additionally, *ortho*-bromo and *ortho*-chloro phenylalanine was substituted into lispro insulin at position B24. These analogues are designated 2Br-$Phe^{B24}$-KP-insulin and 2Cl-$Phe^{B24}$-KP-insulin, respectively. Designations of respective analogues of KP-insulin and DKP-insulin are abbreviated in tables and figures as KP and DKP with "insulin" omitted for brevity.

More specifically, for halogenated phenylalanine substitutions at B24, a synthetic octapeptide representing residues (N)-GF*FYT<u>KP</u>T (halogenated phenylalanine residue indicated as "F*" and "KP" substitutions (underlined); SEQ ID NO. 9) and truncated analogue *des*-octapeptide[B23-B30]-insulin (wild type at position B10, SEQ ID NO. 17) were used. For fluorinated phenylalanine substitutions at B25, a synthetic octapeptide representing residues (N)-GFF*YT<u>KP</u>T (fluorinated phenylalanine indicated again as "F*" and "KP" substitutions (underlined); SEQ ID NO. 9) and truncated analogue *des*-octapeptide[B23-B30]-insulin (wild type at position B10; SEQ. ID. NO 17) were used. For fluorinated phenylalanine substitutions at B26, a synthetic octapeptide representing residues (N)-GFFF*T<u>KP</u>T (fluorinated phenylalanine indicated again as "F*" and "KP" substitutions (underlined); SEQ ID NO. 18) and truncated analogue *des*-octapeptide[B23-B30]-insulin (wild type at position B10; SEQ. ID. NO 17) were used. Similarly, for chloro- and bromo-phenylalanine substitutions at B24 (2Cl and 2Br), a synthetic octapeptide representing residues (N)-GF*FYT<u>KP</u>T (halogenated phenylalanine residue indicated as "F*" and "KP" substitutions (underlined); SEQ ID NO. 9) and truncated analogue *des*-octapeptide[B23-B30]-insulin (wild type at position B10, SEQ ID NO. 17) were used. Typically, *des*-octapeptide (15 mg) and octapeptide (15 mg) were dissolved in a mixture of dimethylacetamide/1,4-butandiol/0.2 M Tris acetate (pH 8) containing 10 mM calcium acetate and 1 mM ethylene diamine tetra-acetic acid (EDTA) (35:35:30, v/v, 0.4 mL). The final pH was adjusted to 7.0 with 10 μL of N-methylmorpholine. The solution was cooled to 12° C, and 1.5 mg of TPCK-trypsin was added and incubated for 2 days at 12° C. An additional 1.5 mg of trypsin was added after 24 hr. The reaction was acidified with 0.1% trifluoroacetic acid and purified by preparative reverse-phase HPLC (C4). Mass spectrometry using matrix-assisted laser desorption/ionization time-of-flight (MALDI-TOF; Applied Biosystems, Foster City, CA) in each case gave expected values. Relative activity and dissociation constants were determined as described above.

The resulting data is presented in Table 2 for substitution of a fluorinated phenylalanine at position B24 of insulin. For comparison purposes, wild type insulin and 2F-Phe$^{B24}$-DKP-insulin were tested under the same conditions at 37° C. Data for testing at 37° C are presented below in Table 3. Data for substitutions of fluorinated phenylalanine at positions B24, B25 and B26 and bromo- and chloro- substituted phenylalanine in a lispro insulin analogue background are presented below in Table 4.

**Table 2**

Stability and Activity of Phe$^{B24}$-DKP

| Sample | $\Delta G_u$ (Kcal/mol) | $\Delta\Delta G_u$ (Kcal/mol) | $C_{mid}$ (M Gu-HCl) | *m* (Kcal/mol/M) | receptor binding |
|---|---|---|---|---|---|
| DKP-insulin | 4.3 ± 0.06 | / | 5.4 ± 0.1 | 0.80 ± 0.01 | 161 |
| 2F-Phe$^{B24}$-DKP | 4.9 ± 0.1 | 0.6 | 5.8 ± 0.1 | 0.85 ± 0.02 | 37 |
| 3F-Phe$^{B24}$-DKP | 3.8 ± 0.1 | -0.5 | 5.5 ± 0.2 | 0.70 ± 0.02 | 102 |
| 4F-Phe$^{B24}$-DKP | 3.9 ± 0.1 | -0.4 | 5.6 ± 0.2 | 0.70 ± 0.02 | 43 |
| F5- Phe$^{B24}$-DKP | 4.84 ± 0.1 | 0.54 | 5.8 ± 0.1 | 0.85 ± 0.02 | 1 |

**Table 3**

Stability and Activity of Insulin and Phe$^{B24}$-DKP-insulin [37°C]

| Sample | $\Delta G_u$ (Kcal/mol) | $\Delta\Delta G_u$ (Kcal/mol) | $C_{mid}$ (M Gu-HCl) | *m* (Kcal/mol/M) | receptor binding |
|---|---|---|---|---|---|
| wt-insulin | 2.4 ± 0.10 | - | 4.1 ± 0.17 | 0.57 ± 0.06 | 100 |
| 2F-Phe$^{B24}$-DKP | 3.1 ± 0.1 | 0.7 | 4.9 ± 0.1 | 0.64 ± 0.03 | |

**Table 4**

Stability And Activity Of Halogenated-Phe Analogue Of Lispro Insulin

| receptor Sample | $\Delta G_u$ (Kcal/mol) | $\Delta\Delta G_u$ (Kcal/mol) | $C_{mid}$ (M Gu-HCl) | *m* (Kcal/mol/M) | binding |
|---|---|---|---|---|---|
| KP-insulin | 3.0 ± 0.06 | / | 4.5 ± 0.1 | 0.61 ± 0.01 | 92 |
| 2F-Phe$^{B24}$-KP | 3.8 ± 0.1 | 1.0 ± 0.16 | 4.9 ± 0.1 | 0.76 ± 0.02 | 17 |
| 3F-Phe$^{B24}$-KP | 3.0 ± 0.1 | 0.2 ± 0.16 | 4.5 ± 0.15 | 0.67 ± 0.02 | 12 |
| 4F-Phe$^{B24}$-KP | 2.75 ± 0.1 | 0.05 ± 0.16 | 4.4 ± 0.1 | 0.62 ± 0.02 | 32 |
| $F_5$-Phe$^{B24}$-KP | 3.8 ± 0.1 | 1.0 ± 0.16 | 5.0 ± 0.1 | 0.76 ± 0.01 | 0.04 |
| 2F-Phe$^{B25}$-KP | 3.3 ± 0.1 | 0.5 ± 0.16 | 4.3 ± 0.2 | 0.76 ± 0.03 | 15 |
| 3F-Phe$^{B25}$-KP | 2.9 ± 0.1 | 0.1 ± 0.16 | 4.5 ± 0.2 | 0.65 ± 0.02 | 41 |
| 4F-Phe$^{B25}$-KP | 2.9 ± 0.1 | 0.1 ± 0.16 | 4.5 ± 0.1 | 0.65 ± 0.01 | 78 |
| $F_5$-Phe$^{B25}$-KP | 2.9 ± 0.1 | 0.1 ± 0.16 | 4.5 ± 0.2 | 0.65 ± 0.03 | 0.4 |
| 2F-Phe$^{B26}$-KP | 1.7 ± 0.1 | -1.1 ± 0.16 | 4.0 ± 0.2 | 0.43 ± 0.03 | 0.1 |
| 3F-Phe$^{B26}$-KP | 3.3 ± 0.1 | 0.5 ± 0.16 | 4.86 ± 0.2 | 0.69 ± 0.03 | 17 |
| 4F-Phe$^{B26}$-KP | 3.7 ± 0.1 | 0.9 ± 0.16 | 4.9 ± 0.2 | 0.75 ± 0.02 | 13 |
| F5-Phe$^{B26}$-KP | 3.6 ± 0.2 | 0.8 ± 0.26 | 4.7 ± 0.26 | 0.76 ± 0.04 | 3 |
| 2Br-Phe$^{B24}$-KP | 3.6 ± 0.05 | 0.8 ± 0.11 | 4.8 ± 0.1 | 0.75 ± 0.01 | 31 |
| 2Cl-Phe$^{B24}$-KP | 3.8 ± 0.06 | 1.0 ± 0.12 | 4.9 ± 0.1 | 0.77 ± 0.01 | 36 |

Receptor-binding data for analogues of lispro insulin by competitive displacement are also presented in Fig. 6. The assay employed the B-isoform of the insulin receptor and $^{125}$I-Tyr$^{A14}$-human insulin as tracer.

Lispro insulin analogues containing either chloro- or bromo-substituted phenylalanine at B24 (2Cl-Phe$^{B24}$-KP-insulin and 2Br-Phe$^{B24}$-KP-insulin, respectively) were tested for the ability to lower blood sugar in diabetic rats in comparison to non-halogenated lispro (KP) insulin. Male Lewis rats (~250 g body weight) were rendered diabetic with streptozotocin. Lispro insulin analogue, 2Cl-Phe$^{B24}$-KP-insulin and 2Br-Phe$^{B24}$-KP-insulin were purified by HPLC, dried to powder, and dissolved in insulin diluent (Eli Lilly Corp). Rats were injected subcutaneously at time = 0 with 20 µg or 6.7 µg insulin analogue in 100 µl of diluent. Blood was obtained from clipped tip of the tail at time 0 and every 10 minutes up to 90 min. Blood glucose was measured using a Hypoguard Advance Micro-Draw meter. Blood glucose concentration changes (in milligrams (mg) per decaliter (dL) per hour (h)) are listed in Tables 5 and 6 (these studies were performed with different sets of rats and so exhibit differences in baseline pharmacodynamic response to control injections of KP-insulin; top lines in each table).

**Table 5**

Blood Glucose Levels In Response To Halogenated Analogues Of Lispro Insulin

| Sample | Insulin Analogue (μg) | Δ Blood Glucose (mg/dL/h) |
|---|---|---|
| KP-insulin | 20 | 191 +/- 20 |
| KP-insulin | 6.7 | 114 +/- 15 |
| 2Cl-Phe$^{B24}$-KP | 20 | 194 +/- 26 |
| 2Cl-Phe$^{B24}$-KP | 6.7 | 179 +/- 30 |
| 2Br-Phe$^{B24}$-KP | 20 | 224 +/- 39 |
| 2Br-Phe$^{B24}$-KP | 6.7 | 189 +/- 13 |

**Table 6**

Blood Glucose Levels In Response To Halogenated Insulin Analogues

| Sample | Insulin Analogue (μg) | Δ Blood Glucose (mg/dL/h) |
|---|---|---|
| KP-insulin | 6.7 | 164 +/- 20 |
| 2F-Phe$^{B24}$-DKP | 6.7 | 180+/- 8 |
| 2F-Phe$^{B24}$-KP | 6.7 | 131 +/- 16 |
| 4F-Phe$^{B26}$-KP | 6.7 | 164 +/- 17 |

As seen in Tables 5 and 6, the halogenated-phenylalanine containing insulin analogues were equal or greater in potency than lispro (Humalog®) insulin with the exception of 2F-Phe$^{B24}$-KP-insulin. The potency of a 2F-Phe$^{B24}$ insulin analogue can be restored to that of KP-insulin by inclusion of the Asp$^{B10}$ substitution in the 2F-Phe$^{B24}$-DKP-insulin analogue (line 2 of Table 6). Additionally, the presence of a halogenated phenylalanine at position B24 increases fibrillation lag time between three-fold and four-fold; thus, whereas KP-insulin under the above experimental conditions exhibits a lag time of about 3 days, substitution of Phe$^{B24}$ by either 2F-Phe$^{B24}$, 2Cl-Phe$^{B24}$, or 2Br-Phe$^{B24}$ extends the lag time to between 10-12 days. These modifications also increase the thermodynamtic stability (as probed by guanidine denaturation) by between 0.5 and 1 kcal/mol (Table 4).

Dissociation constants ($K_d$) were determined as described by Whittaker and Whittaker (2005. J. Biol. Chem. 280: 20932-20936), by a competitive displacement assay using $^{125}$I-Tyr$^{A14}$-insulin (kindly provided by Novo-Nordisk) and the purified and solubilized insulin receptor (isoform B) in a microtiter plate antibody capture assay with minor modification; transfected receptors were tagged at their C-terminus by a triple repeat of the FLAG epitope (DYKDDDDK; SEQ. ID. NO 11) and microtiter plates were coated by anti-FLAG M2 monoclonal antibody (Sigma). The percentage of tracer bound in the absence of competing ligand was less than 15% to avoid ligand-depletion artifacts. Binding data were analyzed by non-linear regression using a heterologous competition model (Wang, 1995, FEBS Lett. 360: 111-114) to obtain dissociation constants.

**Table 7**

Cross-Binding of Insulin Analogues to the IGF Receptor

| protein | $K_d$ (nM) | relative to insulin |
|---|---|---|
| IGF-I | 0.037 ± 0.003 | 260 |
| human insulin | 9.6 ± 0.3 | 1 |
| KP-insulin | 10.6 ± 1 | 1 |
| Asp$^{B10}$-insulin | 4.2 | 2 |
| Arg$^{B31}$, Arg$^{B32}$, Gly$^{A21}$-insulin | 3.1 | 3 |
| DKP-insulin | 1.6 ± 0.1 | 6 |
| 2F-Phe$^{B24}$-KP | 34 | < 0.3 |
| 2Br-Phe$^{B24}$-KP | 10.1 | 1 |
| 2Cl-Phe$^{B24}$-KP | 9.6 | 1 |
| 2F-Phe$^{B24}$-DKP | 9.2 | 1 |

Measurements of cross-binding of selected insulin analogues to the IGF receptor are summarized in Table 7. Human insulin under these conditions binds to IGFR 260-fold less tightly than does IGF-I. Cross-binding of $Asp^{B10}$-insulin is increased by twofold whereas cross-binding by an analogue in wide clinical use, $Arg^{B31}$, $Arg^{B32}$, $Gly^{A21}$-insulin (insulin glargine™; Lantus™), is increased by threefold. Such augmentation of IGFR cross-binding is undesirable as treatment of Sprague-Dawley rats with $Asp^{B10}$-insulin is associated with an increased incidence of mammary tumors whereas recent clinical studies suggest the possibility that human use of Lantus confers a dose-dependent increase in the risk of diverse cancers. (The sequence of IGF-I contains Glu at position B10, similar in negative charge to $Asp^{B10}$. While not wishing to be bound by theory, it is believed that cross-binding by Lantus is augmented by the two basic residues at the C-terminus of the B-chain. Because IGF-I also contains Lys at position B28 and Pro at position B29, it is possible that KP-insulin may exhibit a small increase in cross-binding but the above data are not sufficiently precise to establish this.) DKP-insulin exhibits a six-fold increase in IGFR cross-binding. Significantly, this increase is counter-balanced by an *ortho*-monofluoro substitution at B24: 2F-$Phe^{B24}$-DKP-insulin exhibits the same low level of cross-binding as wild-type human insulin. Its hypoglycemic potency in diabetic rats is also the same as that of human insulin (Table 6). Because 2F-$Phe^{B24}$-DKP-insulin is monomeric even at protein concentrations > 0.5 mM, this analogue is therefore of potential utility as an ultra-fast acting and ultra-stable insulin formulation for clinical use. Cross-binding affinities of 2Cl-$Phe^{B24}$-KP-insulin and 2Br-$Phe^{B24}$-KP-insulin are also at a low level similar to that of human insulin. These analogues are also fully active in diabetic rats (Table 5).

CD spectra were determined for human insulin, KP-insulin (lispro) and halogenated $Phe^{B24}$ insulin analogues as described above. CD-detected protein denaturation is provided as a function of the concentration of guanidine hydrochloride in Figs. 7A and 7B. Fig. 7A provides a comparison of human insulin (solid line) and KP-insulin (dashed line; "parent") with KP-insulin analogues containing monofluorous substitution of $Phe^{B24}$ at position 2, 3, or 4 (filled triangles, filled squares, and open circles, respectively). Fig. 7B provides a comparison of human insulin (solid line) and KP-insulin (dashed line; "parent") with analogues containing 2-Cl or 2-Br modification of $Phe^{B24}$ (filled inverted triangles and open triangles, respectively). Inferred thermodynamic parameters are provided above in Table 4. Fractional unfolding was monitored by mean residue ellipticity at 222 nm at 25° C.

NMR structures of selected insulin analogues have been obtained to demonstrate that halogen substitutions are well accommodated in insulin and do not cause transmitted conformational perturbations. $^1$H-NMR spectra are provided in Figs. 8A and 8B. The NMR structure of 2Cl-$Phe^{B24}$-KP-insulin as a monomer in solution is thus similar to that of KP-insulin; similarly, the NMR structure of 2F-$Phe^{B24}$-DKP-insulin (also as a monomer in solution) is similar to that of DKP-insulin. Qualitative analysis of NMR spectra of 2F-$Phe^{B24}$-KP-insulin and 2Br-$Phe^{B24}$ -KP-insulin likewise indicate native-like structures.

Crystal structures have been determined of 2F-$Phe^{B24}$-KP-insulin and 4F-$Phe^{B24}$-KP-insulin as zinc-stabilized hexamers in the presence of the preservative phenol (data not shown). The structures are similar to those previously reported for KP-insulin as a zinc-stabilized hexamer in the presence of phenol. In each case the conformation of the hexamer is $T_3R^f_3$ containing two axial zinc ions and three bound phenol molecules. Electron density for the halogen atoms is readily observed. The structure of the dimer- and trimer-forming surfaces are preserved in the halogenated analogues, suggesting that pharmaceutical formulations may be obtained similar to those employed for KP-insulin and other fast-acting insulin analogues.

A method for treating a patient comprises administering a halogen-substituted insulin analogue to the patient. In one example, the halogen-substituted insulin analogue is an insulin analogue containing halogen-substituted phenylalanine, such as a fluoro-, chloro- or bromo-substituted phenylalanine. In one particular example the halogen substituted phenylalanine is 2F-$Phe^{B24}$, 2F-$Phe^{B25}$, or 2F-$Phe^{B26}$. In another example, the halogen-substituted insulin analogue additionally contains one or more substitutions elsewhere in the insulin molecule designed to alter the rate of action of the analogue in the body. In still another example, the insulin analogue is administered by an external or implantable insulin pump. An insulin analogue of the present invention may also contain other modifications, such as a tether between the C-terminus of the B-chain and the N-terminus of the A-chain as described more fully in co-pending International Application No. PCT/US07/080467, the disclosure of which is incorporated by reference herein.

A pharamaceutical composition may comprise such insulin analogues and which may optionally include zinc. Zinc ions may be included in such a composition at a level of a molar ratio of between 2.2 and 3.0 per hexamer of the insulin analogue. In such a formulation, the concentration of the insulin analogue would typically be between about 0.1 and about 3 mM; concentrations up to 3 mM may be used in the reservoir of an insulin pump. Modifications of meal-time insulin analogues may be formulated as described for (a) "regular" formulations of Humulin™ (Eli Lilly and Co.), Humalog™ (Eli Lilly and Co.), Novalin™ (Novo-Nordisk), and Novalog™ (Novo-Nordisk) and other rapid-acting insulin formulations currently approved for human use, (b) "NPH" formulations of the above and other insulin analogues, and (c) mixtures of such formulations.

Excipients may include glycerol, glycine, other buffers and salts, and anti-microbial preservatives such as phenol and *meta*-cresol; the latter preservatives are known to enhance the stability of the insulin hexamer. Such a pharmaceutical composition may be used to treat a patient having diabetes mellitus or other medical condition by administering a physiologically effective amount of the composition to the patient.

A nucleic acid comprising a sequence that encodes a polypeptide encoding an insulin analogue containing a sequence encoding at least a B-chain of insulin with a fluorinated phenylalanine at position B24, B25 or B26 is also envisioned. This can be accomplished through the introduction of a stop codon (such as the amber codon, TAG) at position B24 in conjunction with a suppressor tRNA (an amber suppressor when an amber codon is used) and a corresponding tRNA synthetase, which incorporates a non-standard amino acid into a polypeptide in response to the stop codon, as previously described (Furter, 1998, Protein Sci. 7:419-426; Xie et al., 2005, Methods. 36: 227-238). The particular sequence may depend on the preferred codon usage of a species in which the nucleic acid sequence will be introduced. The nucleic acid may also encode other modifications of wild-type insulin. The nucleic acid sequence may encode a modified A- or B-chain sequence containing an unrelated substitution or extension elsewhere in the polypeptide or modified proinsulin analogues. The nucleic acid may also be a portion of an expression vector, and that vector may be inserted into a host cell such as a prokaryotic host cell like an *E. coli* cell line, or a eukaryotic cell line such as *S. cereviciae* or *Pischia pastoris* strain or cell line.

For example, it is envisioned that synthetic genes may be synthesized to direct the expression of a B-chain polypeptide in yeast *Piscia pastoris* and other microorganisms. The nucleotide sequence of a B-chain polypeptide utilizing a stop codon at position B24 for the purpose of incorporating a halogenated phenylalanine at that position may be either of the following or variants thereof:

(a) with Human Codon Preferences:

TTTGTGAACCAACACCTGTGCGGCTCACACCTGGTGGAAGCTCTCTACCTAGTGTGC
GGGGAACGAGGCTAGTTCTACACACCCAAGACC (SEQ. ID. NO.12)

(b) with Pichia Codon Preferences:

TTTGTTAACCAACATTTGTGTGGTTCTCATTTGGTTGAAGCTTTGTACTTGGTTTGTG
GTGAAAGAGGTTAGTTTTACACTCCAAAGACT (SEQ. ID. NO.13)

Similarly, a full length pro-insulin cDNA having human codon preferences and utilizing a stop codon at position B24 for the purpose of incorporating a halogenated phenylalanine at that position may have the sequence of SEQ. ID NO. 14.

TTTGTGAACC AACACCTGTG CGGCTCACAC CTGGTGGAAG CTCTCTACCT
AGTGTGCGGG GAACGAGGCT AGTTCTACAC ACCCAAGACC CGCCGGGAGG
CAGAGGACCT GCAGGTGGGG CAGGTGGAGC TGGGCGGCGG CCCTGGTGCA
GGCAGCCTGC AGCCCTTGGC CCTGGAGGGG TCCCTGCAGA AGCGTGGCAT
TGTGGAACAA TGCTGTACCA GCATCTGCTC CCTCTACCAG CTGGAGAACT
ACTGCAACTA G (SEQ.ID. NO.14)

Likewise, a full length human pro-insulin cDNA utilizing a stop codon at position B24 for the purpose of incorporating a halogenated phenylalanine at that position and having codons preferred by *P. pastoris* may have the sequence of SEQ. ID. NO 15

TTTGTTAACC AACATTTGTG TGGTTCTCAT TTGGTTGAAG CTTTGTACTT
GGTTTGTGGT GAAAGAGGTT AGTTTTACAC TCCAAAGACT AGAAGAGAAG
CTGAAGATTT GCAAGTTGGT CAAGTTGAAT TGGGTGGTGG TCCAGGTGCT
GGTTCTTTGC AACCATTGGC TTTGGAAGGT TCTTTGCAAA AGAGAGGTAT
TGTTGAACAA TGTTGTACTT CTATTTGTTC TTTGTACCAA TTGGAAAACT
ACTGTAACTA A (SEQ. ID. NO. 15)

Similarly, a nucleotide sequence of a B-chain polypeptide utilizing a stop codon at position B25 for the purpose of incorporating a halogenated phenylalanine at that position may be either of the following or variants thereof:

(b) with Human Codon Preferences:

TTTGTGAACCAACACCTGTGCGGCTCACACCTGGTGGAAGCTCTCTACCTAGTGTGC
GGGGAACGAGGCTTCTAGTACACACCCAAGACC (SEQ. ID. NO.19)

(b) with Pichia Codon Preferences:

TTTGTTAACCAACATTTGTGTGGTTCTCATTTGGTTGAAGCTTTGTACTTGGTTTGTG
GTGAAAGAGGTTTTTAGTACACTCCAAAGACT (SEQ. ID. NO.20)

A full length pro-insulin cDNA having human codon preferences and utilizing a stop codon at position B25 for the purpose of incorporating a halogenated phenylalanine such as fluorinated phenylalanine at that position may have the sequence of SEQ. ID NO. 21.

TTTGTGAACC AACACCTGTG CGGCTCACAC CTGGTGGAAG CTCTCTACCT
AGTGTGCGGG GAACGAGGCT TCTAGTACAC ACCCAAGACC CGCCGGGAGG
CAGAGGACCT GCAGGTGGGG CAGGTGGAGC TGGGCGGCGG CCCTGGTGCA
GGCAGCCTGC AGCCCTTGGC CCTGGAGGGG TCCCTGCAGA AGCGTGGCAT
TGTGGAACAA TGCTGTACCA GCATCTGCTC CCTCTACCAG CTGGAGAACT
ACTGCAACTA G (SEQ.ID. NO.21)

Likewise, a full length human pro-insulin cDNA utilizing a stop codon at position B25 for the purpose of incorporating a halogenated phenylalanine such as a fluorinated phenylalanine at that position and having codons preferred by *P. pastoris* may have the sequence of SEQ. ID. NO 22.

TTTGTTAACC AACATTTGTG TGGTTCTCAT TTGGTTGAAG CTTTGTACTT
GGTTTGTGGT GAAAGAGGTT TTTAGTACAC TCCAAAGACT AGAAGAGAAG
CTGAAGATTT GCAAGTTGGT CAAGTTGAAT TGGGTGGTGG TCCAGGTGCT
GGTTCTTTGC AACCATTGGC TTTGGAAGGT TCTTTGCAAA AGAGAGGTAT
TGTTGAACAA TGTTGTACTT CTATTTGTTC TTTGTACCAA TTGGAAAACT
ACTGTAACTA A (SEQ. ID. NO. 22)

Likewise, a nucleotide sequence of a B-chain polypeptide utilizing a stop codon at position B26 for the purpose of incorporating a halogenated phenylalanine at that position may be either of the following or variants thereof:
(c) with Human Codon Preferences:

TTTGTGAACCAACACCTGTGCGGCTCACACCTGGTGGAAGCTCTCTACCTAGTGTGC
GGGGAACGAGGCTTCTTCTAGACACCCAAGACC (SEQ. ID. NO. 23)

(b) with Pichia Codon Preferences:

TTTGTTAACCAACATTTGTGTGGTTCTCATTTGGTTGAAGCTTTGTACTTGGTTTGTG
GTGAAAGAGGTTTTTTTAGACTCCAAAGACT (SEQ. ID. NO. 24)

A full length pro-insulin cDNA having human codon preferences and utilizing a stop codon at position B26 for the purpose of incorporating a halogenated phenylalanine at that position may have the sequence of SEQ. ID NO. 25.

TTTGTGAACC AACACCTGTG CGGCTCACAC CTGGTGGAAG CTCTCTACCT
AGTGTGCGGG GAACGAGGCT TCTTCTAGAC ACCCAAGACC CGCCGGGAGG
CAGAGGACCT GCAGGTGGGG CAGGTGGAGC TGGGCGGCGG CCCTGGTGCA
GGCAGCCTGC AGCCCTTGGC CCTGGAGGGG TCCCTGCAGA AGCGTGGCAT
TGTGGAACAA TGCTGTACCA GCATCTGCTC CCTCTACCAG CTGGAGAACT
ACTGCAACTA G (SEQ.ID. NO.25)

Likewise, a full length human pro-insulin cDNA utilizing a stop codon at position B26 for the purpose of incorporating a halogenated phenylalanine at that position and having codons preferred by *P. pastoris* may have the sequence of SEQ. ID. NO 26.

TTTGTTAACC AACATTTGTG TGGTTCTCAT TTGGTTGAAG CTTTGTACTT
GGTTTGTGGT GAAAGAGGTT TTTTTAGAC TCCAAAGACT AGAAGAGAAG
CTGAAGATTT GCAAGTTGGT CAAGTTGAAT TGGGTGGTGG TCCAGGTGCT
GGTTCTTTGC AACCATTGGC TTTGGAAGGT TCTTTGCAAA AGAGAGGTAT
TGTTGAACAA TGTTGTACTT CTATTTGTTC TTTGTACCAA TTGGAAAACT
ACTGTAACTA A (SEQ. ID. NO. 26)

Other variants of these sequences, encoding the same polypeptide sequence, are possible, given the synonyms in the genetic code.

Based upon the foregoing disclosure, it should now be apparent that halogen-substituted insulin analogues will carry out the objects set forth hereinabove. Namely, these insulin analogues exhibit enhanced thermodynamic stability, resistance to fibrillation and potency in reducing blood glucose levels. The halogen substituted phenylalanine-containing insulin analogues also have reduced cross-reactivity to insulin-like growth factor (IGFR). It is, therefore, to be understood that any variations evident fall within the scope of the claimed invention and thus, the selection of specific component elements can be determined without departing from the spirit of the invention herein disclosed and described.

The following literature is cited to demonstrate that the testing and assay methods described herein would be understood by one of ordinary skill in the art.

Furter, R., 1998. Expansion of the genetic code: Site-directed p-fluoro-phenylalanine incorporation in Escherichia coli. Protein Sci. 7:419-426.

Merrifield, R.B., Vizioli, L.D., and Boman, H.G. 1982. Synthesis of the antibacterial peptide cecropin A (1-33). Biochemistry 21: 5020-5031.

Mirmira, R.G., and Tager, H.S. 1989. Role of the phenylalanine B24 side chain in directing insulin interaction with its receptor: Importance of main chain conformation. J. Biol. Chem. 264: 6349-6354.

Sosnick, T.R., Fang, X., and Shelton, V.M. 2000. Application of circular dichroism to study RNA folding transitions. Methods Enzymol. 317: 393-409.

Wang, Z.X. 1995. An exact mathematical expression for describing competitive biding of two different ligands to a protein molecule FEBS Lett. 360: 111-114.

Weiss, M.A., Hua, Q.X., Jia, W., Chu, Y.C., Wang, R.Y., and Katsoyannis, P.G. 2000. Hierarchiacal protein "un-design": insulin's intrachain disulfide bridge tethers a recognition $\alpha$-helix. Biochemistry 39: 15429-15440.

Whittaker, J., and Whittaker, L. 2005. Characterization of the functional insulin binding epitopes of the full length insulin receptor. J. Biol. Chem. 280: 20932-20936.

Xie, J. and Schultz, P.G. 2005. An expanding genetic code. Methods. 36: 227-238.

The present invention also relates to the following sections:

§ 1. An insulin analogue comprising a B-chain polypeptide incorporating a halogenated phenylalanine at position B24, B25 or B26.

§ 2. The insulin analogue of § 1, wherein the halogenated phenylalanine is located at position B24.

§ 3. The insulin analogue of § 1, wherein the halogenated phenylalanine is *ortho*-monofluoro-phenylalanine, *ortho*-monobromo-phenylalanine or *ortho*-monochloro-phenylalanine.

§ 4. The insulin analogue of any of § 1-3, wherein the analogue is an analogue of a mammalian insulin

§ 5. The insulin analogue of § 4, wherein the analogue is an analogue of human insulin

§ 6. The insulin analogue of § 4, wherein the B-chain polypeptide comprises an amino acid sequence selected from the group consisting of SEQ. ID. NOS. 4-8 and polypeptides having three or fewer additional amino acid substitutions thereof

§ 7. A nucleic acid encoding an insulin analogue according to § 4.

§ 8. The nucleic acid of § 7, wherein the halogenated phenylalanine is encoded by a stop codon.

§ 9. The nucleic acid of § 8, wherein the stop codon is the nucleic acid sequence TAG.

§ 10. An expression vector comprising the nucleic acid sequence of § 7, 8 or 9.

§ 11. A host cell transformed with the expression vector of § 10.

§ 12. An insulin analogue or a physiologically acceptable salt thereof, wherein the insulin analogue or physiologically acceptable salt thereof contains a B-chain polypeptide incorporating a halogenated phenylalanine at position B24, B25 or B26, for use as a medicament.

§ 13. The insulin analogue or physiologically acceptable salt thereof of § 12, wherein the halogenated phenylalanine is located at position B24.

§ 14. The insulin analogue or physiologically acceptable salt thereof of § 13, wherein the halogenated phenylalanine is *ortho*-monofluoro-phenylalanine, *ortho*-monobromo-phenylalanine or *ortho*-monochloro-phenylalanine.

§ 15. The insulin analogue or physiologically acceptable salt thereof of § 14, wherein the B-chain polypeptide comprises an amino acid sequence selected from the group consisting of SEQ. ID. NOS. 4-8 and polypeptides having three or fewer additional amino acid substitutions thereof.

## Claims

1. A polypeptide comprising an insulin B-chain polypeptide sequence modified by *penta*-fluoro-Phenylalanine at position B24 as determined with reference to the positions of wild type insulin.

2. The polypeptide of claim 1 that is further modified by paired substitutions $Lys^{B28}$ and $Pro^{B29}$.

3. The polypeptide of claim 1 that is further modified by substitution $Asp^{B28}$.

4. The polypeptide of any one of claims 1, 2 or 3, that is further modified by an Asp substitution at position B10.

5. The polypeptide of any one of claims 1-4 comprising an amino acid sequence selected from the group consisting of SEQ. ID. NOS. 4-6 and 8, where the amino acid at position B26 is Tyr, and polypeptides having three or fewer additional amino acid substitutions thereof.

6. The polypeptide of any one of claims 1-4, additionally comprising a His substitution at position A8 relative to wild type insulin.

7. The polypeptide of any of claims 1-4, wherein the insulin B-chain polypeptide sequence is a mammalian insulin B-chain polypeptide sequence.

8. A method of incorporating a *penta*-fluoro-phenylalanine at position B24 of a B-chain polypeptide of an insulin analogue, the method comprising expressing a nucleic acid encoding the insulin analogue, wherein the *penta*-fluoro-phenylalanine is encoded by a stop codon, wherein the nucleic acid is expressed in conjunction with a suppressor tRNA and a corresponding tRNA synthetase which incorporates the non-standard amino acid in response to the stop codon.

**9.** The method of claim 8, wherein the stop codon is the nucleic acid sequence TAG.

**10.** The method of claim 9, wherein the nucleic acid sequence comprises any one of SEQ ID NOS: 12-15.

**11.** The method of any one of claims 8, 9 or 10, wherein the nucleic acid is a portion of an expression vector.

**12.** The method of claim 11, wherein the expression vector is inserted into a host cell.

**13.** An insulin analogue or a physiologically acceptable salt thereof, wherein the insulin analogue or a physiologically acceptable salt thereof comprises an insulin B-chain polypeptide sequence modified by *penta*-fluoro-Phenylalanine at position B24 as determined with reference to the positions of wild type insulin, for use as a medicament.

**14.** The insulin analogue or a physiologically acceptable salt thereof of claim 13, wherein the insulin B-chain polypeptide sequence is further modified by paired substitutions $Lys^{B28}$ and $Pro^{B29}$, or by $Asp^{B28}$.

**15.** The insulin analogue or a physiologically acceptable salt thereof of claim 13 or claim 14, wherein the B-chain polypeptide is further modified by an Asp substitution at position B10.

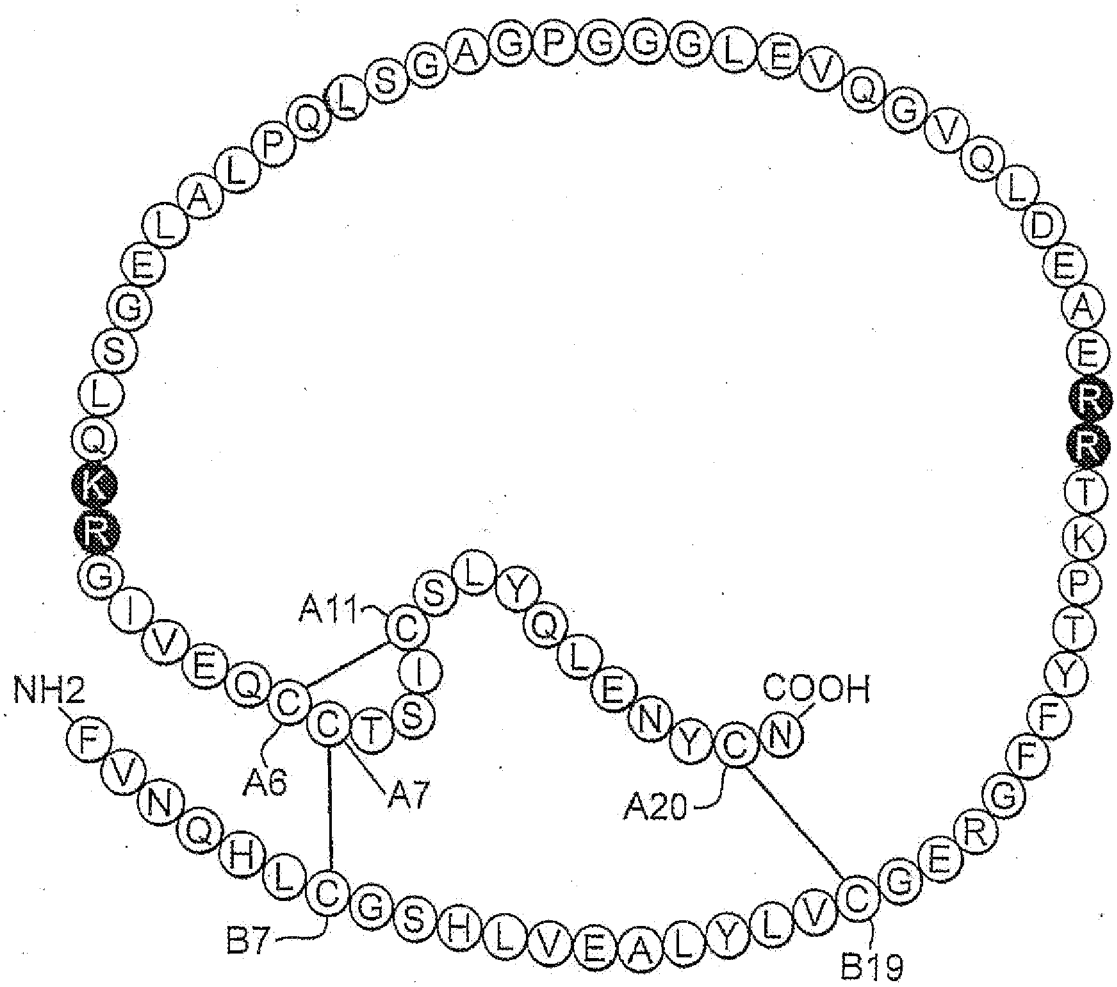


# FIG. 1A

**(PRIOR ART)**
**PROINSULIN**

# FIG. 1B
**(PRIOR ART)**
MODEL

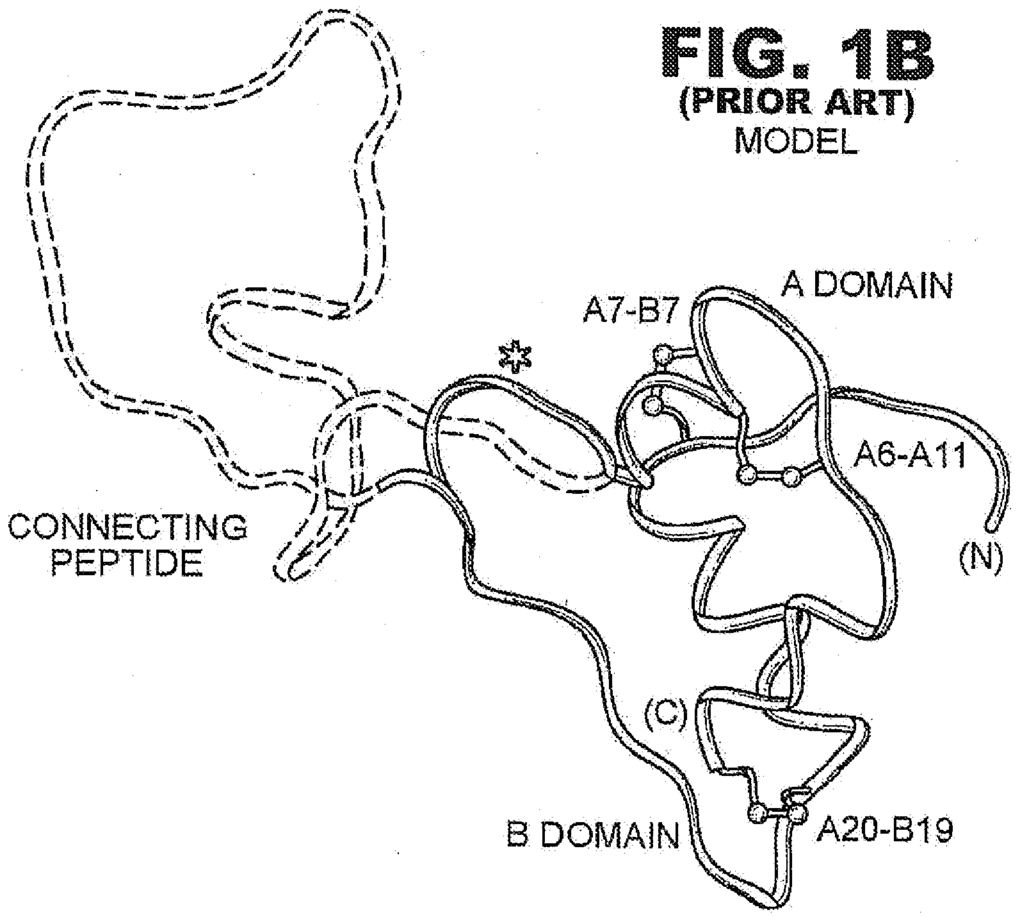

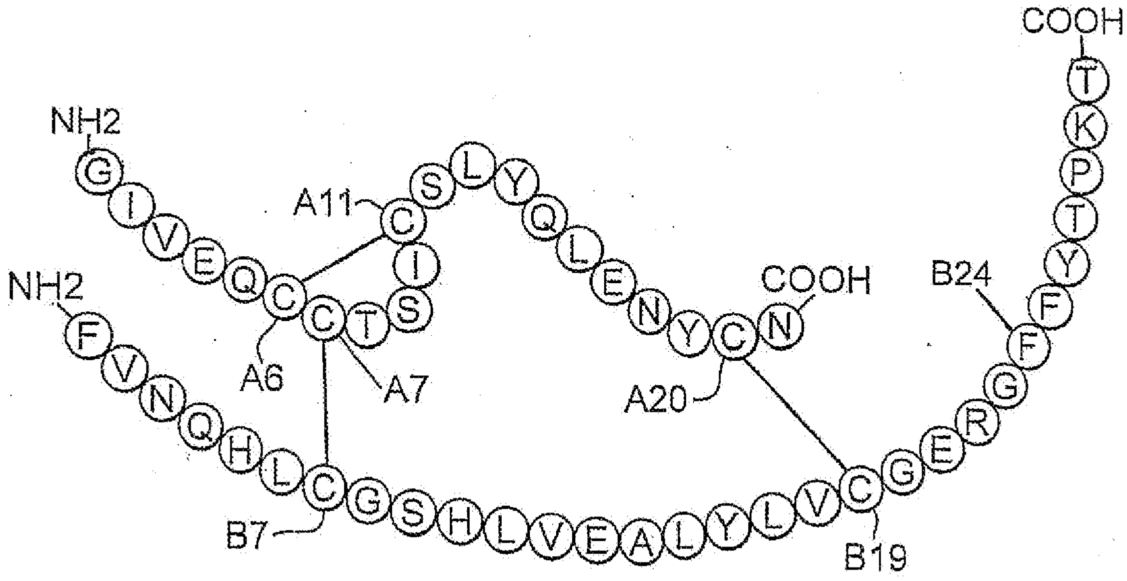


**FIG. 1C**

(PRIOR ART)

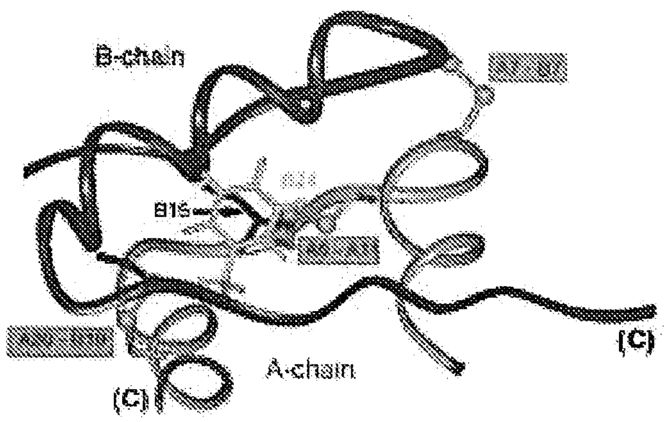


# FIG. 1D

**(PRIOR ART)**

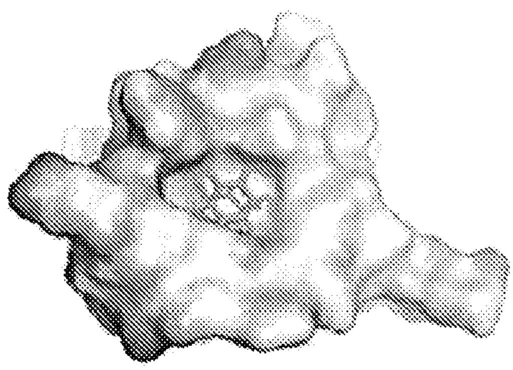

# FIG. 1E

**(PRIOR ART)**

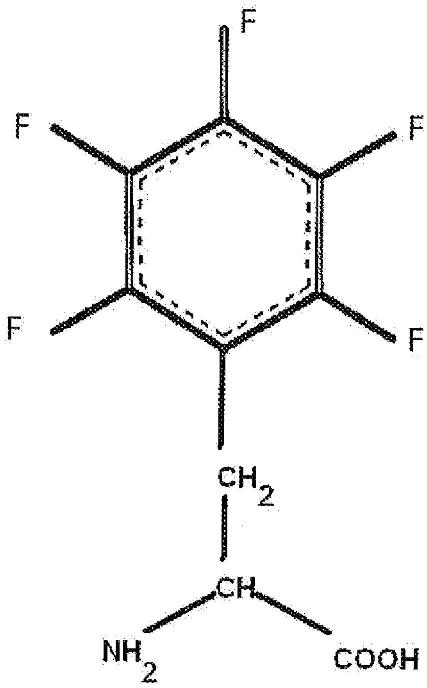

FIG. 2A

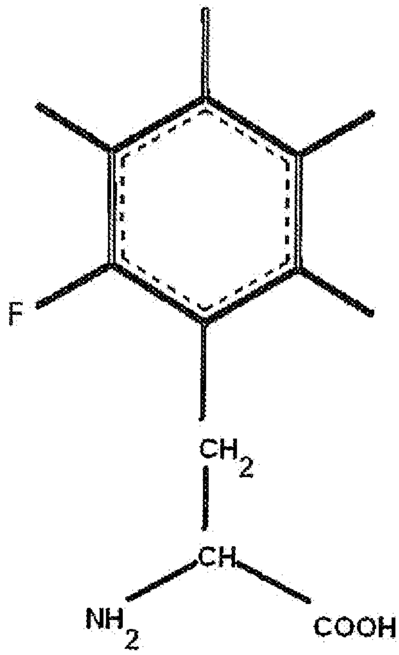

FIG. 2B

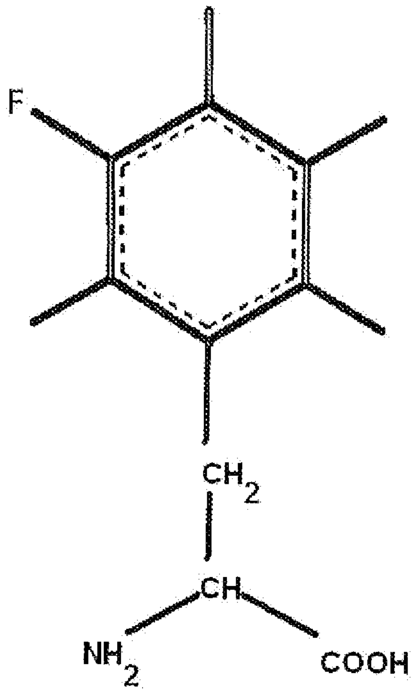

FIG. 2C

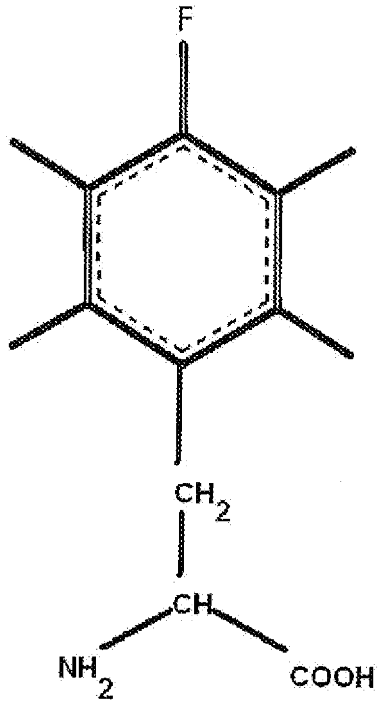

FIG. 2D

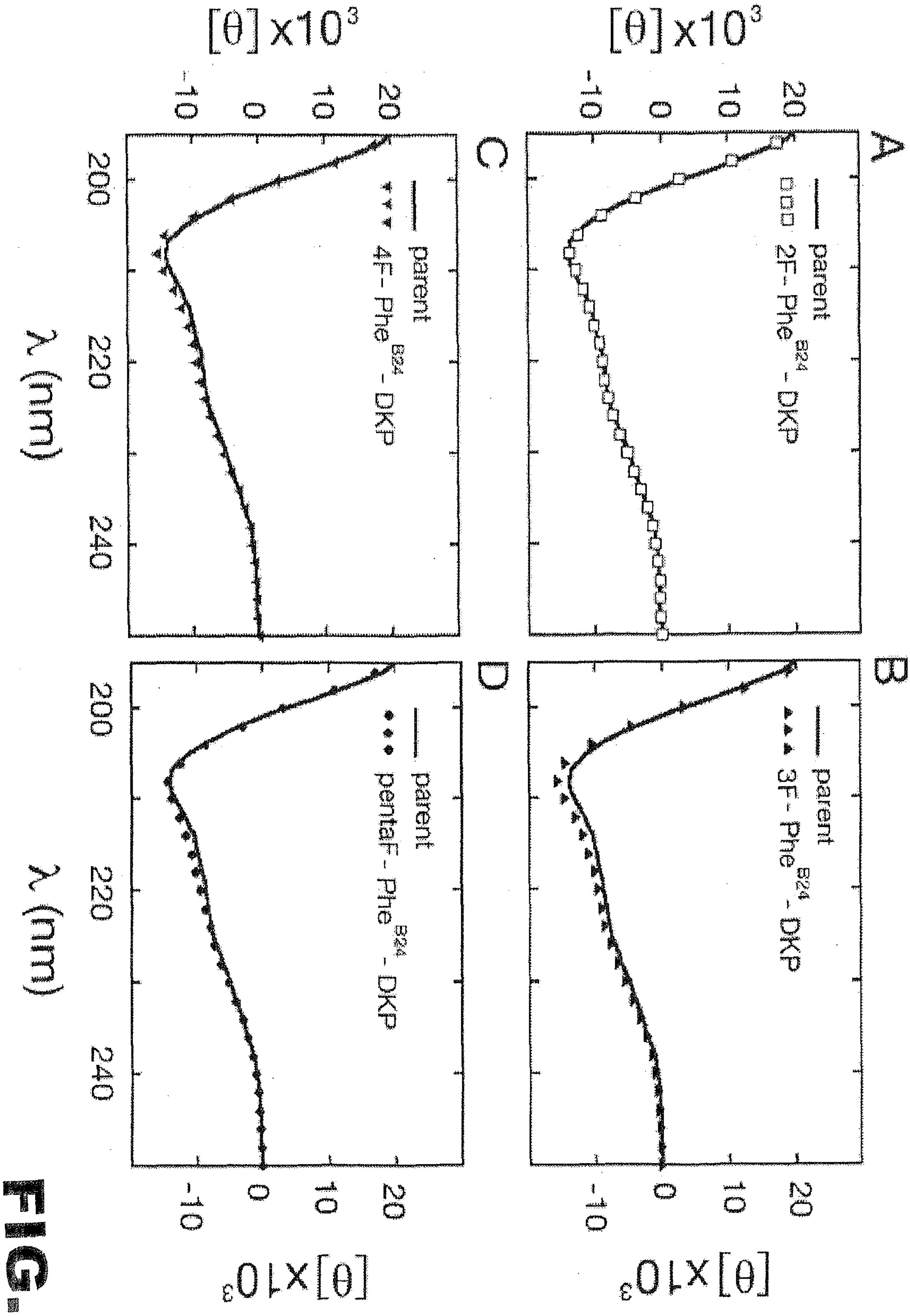
A
parent
2F- Phe$^{B24}$ - DKP
B
parent
3F- Phe$^{B24}$ - DKP
C
parent
4F- Phe$^{B24}$ - DKP
D
parent
pentaF- Phe$^{B24}$ - DKP
[θ] x10$^3$
λ (nm)
200
220
240
-10
0
10
20

FIG. 3

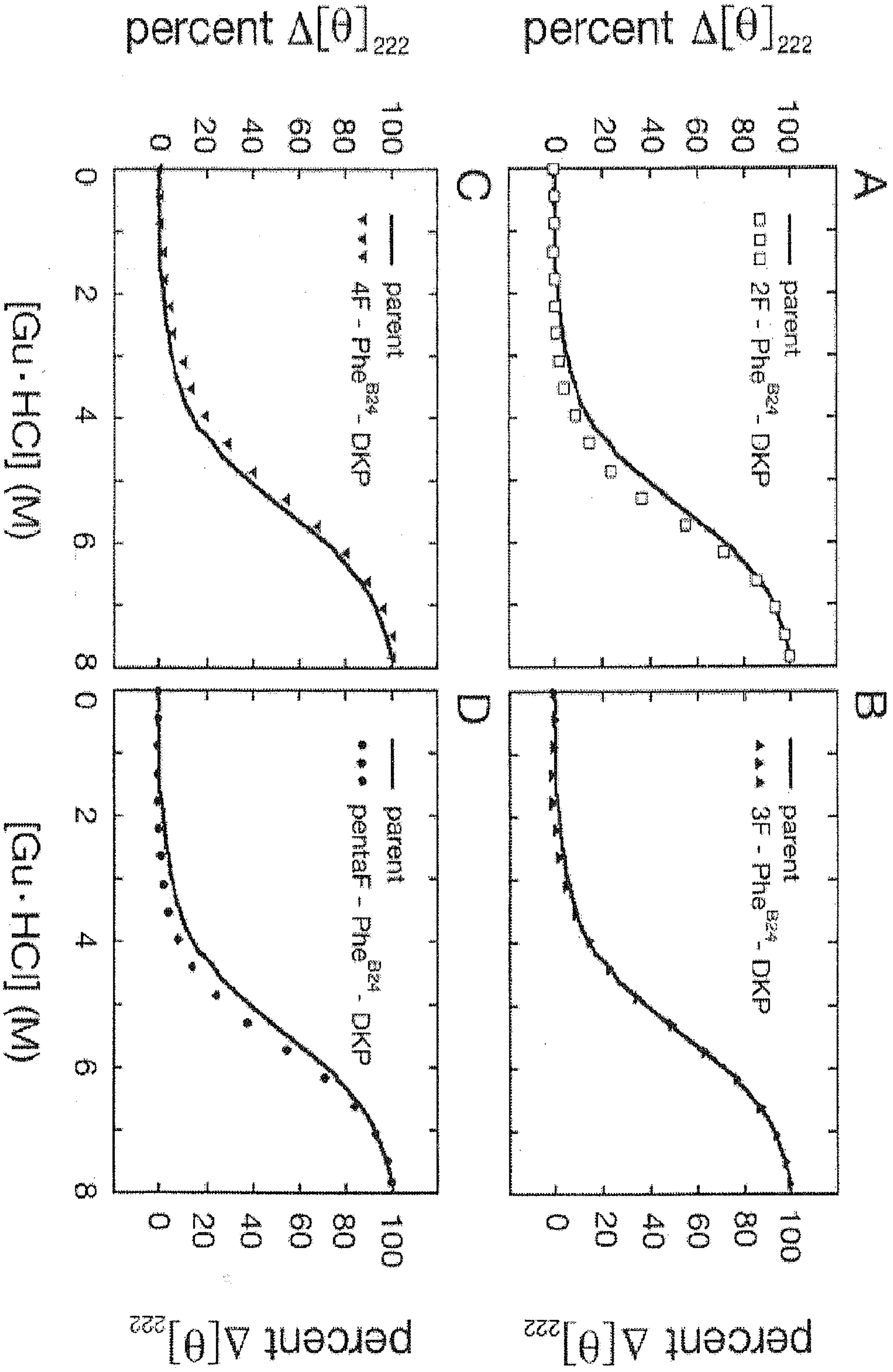
A
parent
2F - PheB24 - DKP
B
parent
3F - PheB24 - DKP
C
parent
4F - PheB24 - DKP
D
parent
pentaF - PheB24 - DKP
percent Δ[θ]222
[Gu·HCl] (M)
0
2
4
6
8
0
20
40
60
80
100

FIG. 4

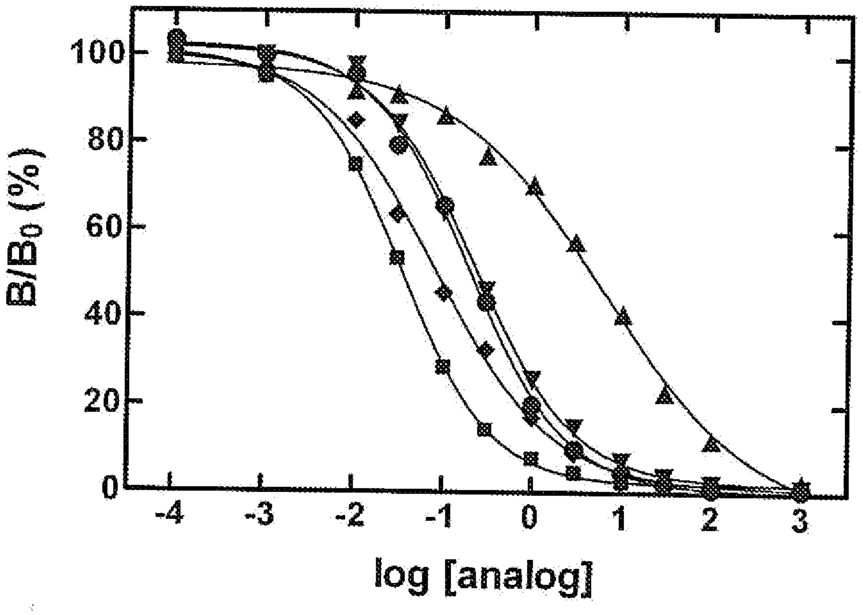


FIG. 5

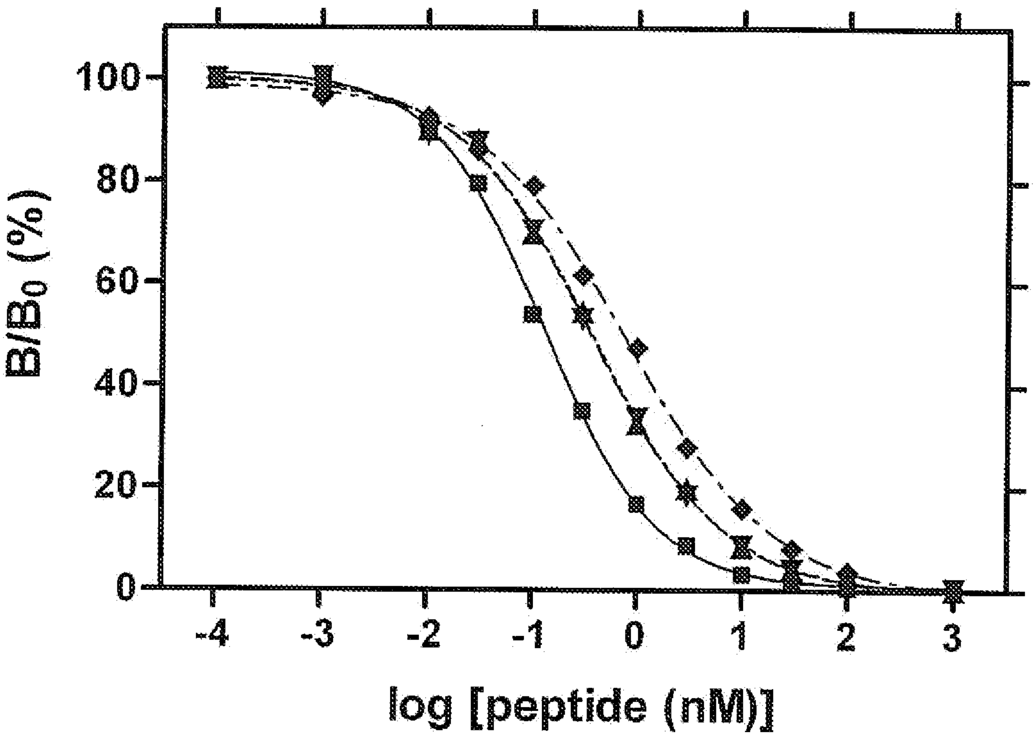


FIG. 6

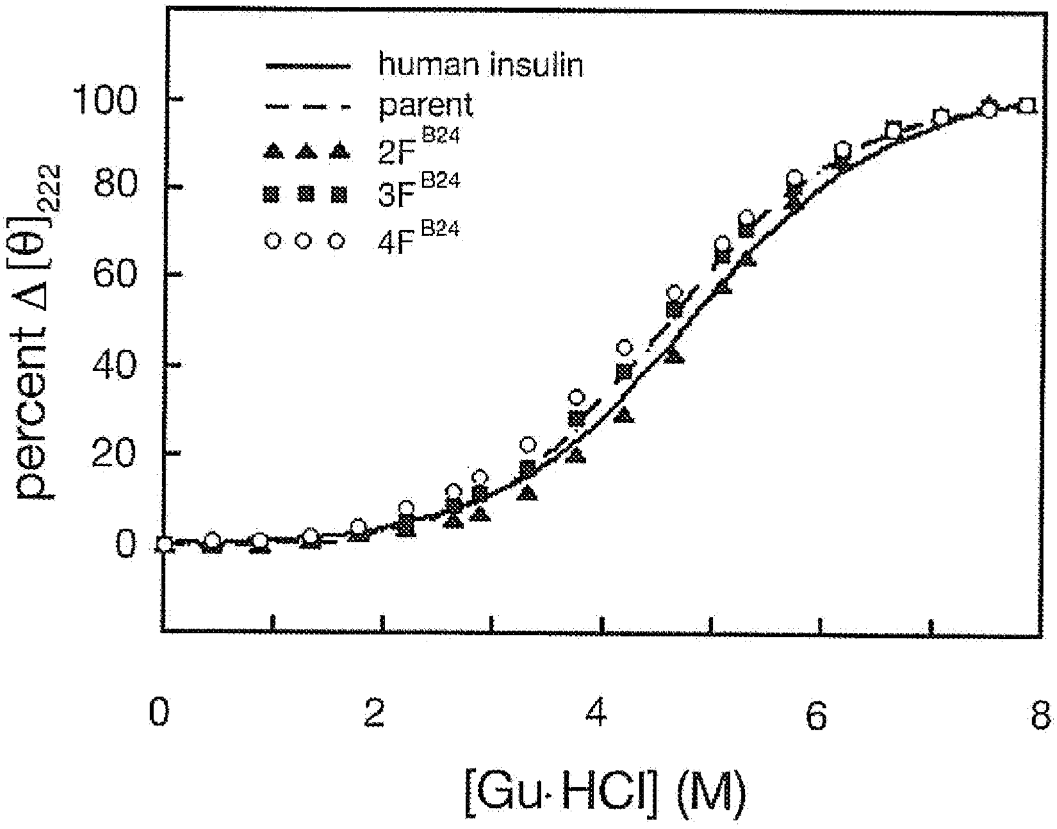
human insulin
parent
2F B24
3F B24
4F B24
percent Δ [θ]222
100
80
60
40
20
0
0
2
4
6
8
[Gu·HCl] (M)

Fig. 7A

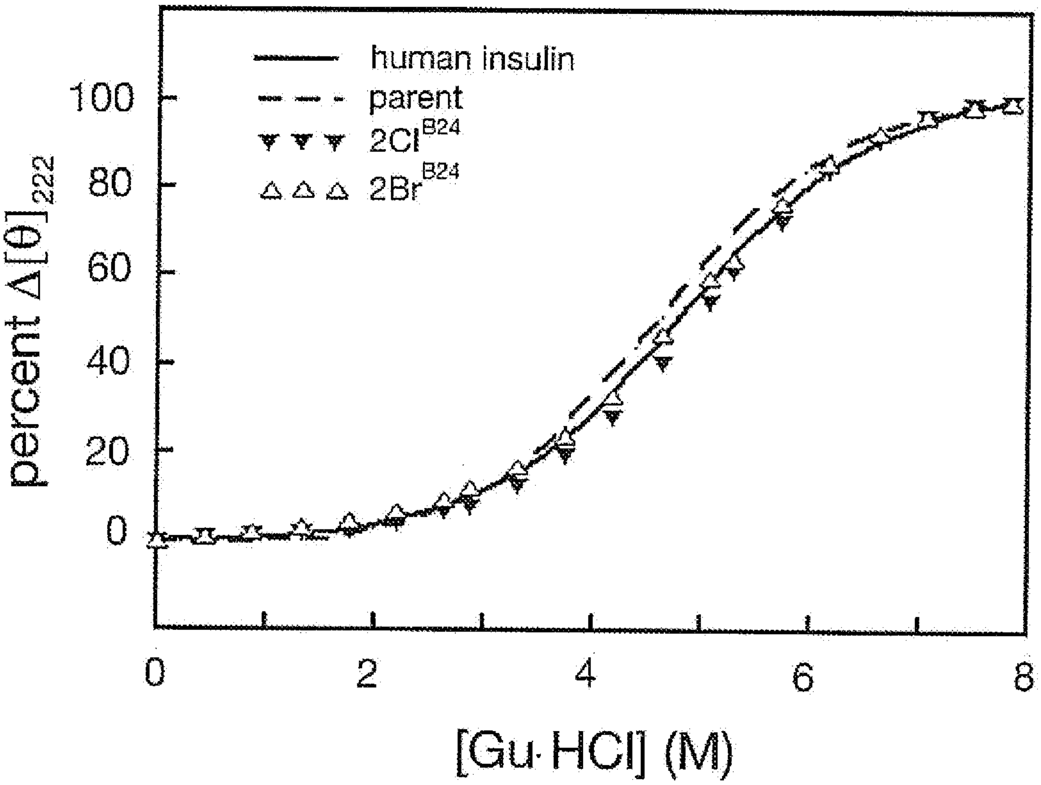

human insulin
parent
2Cl B24
2Br B24
100
80
60
40
20
0
percent Δ[θ]222
0
2
4
6
8
[Gu·HCl] (M)


Fig. 7B

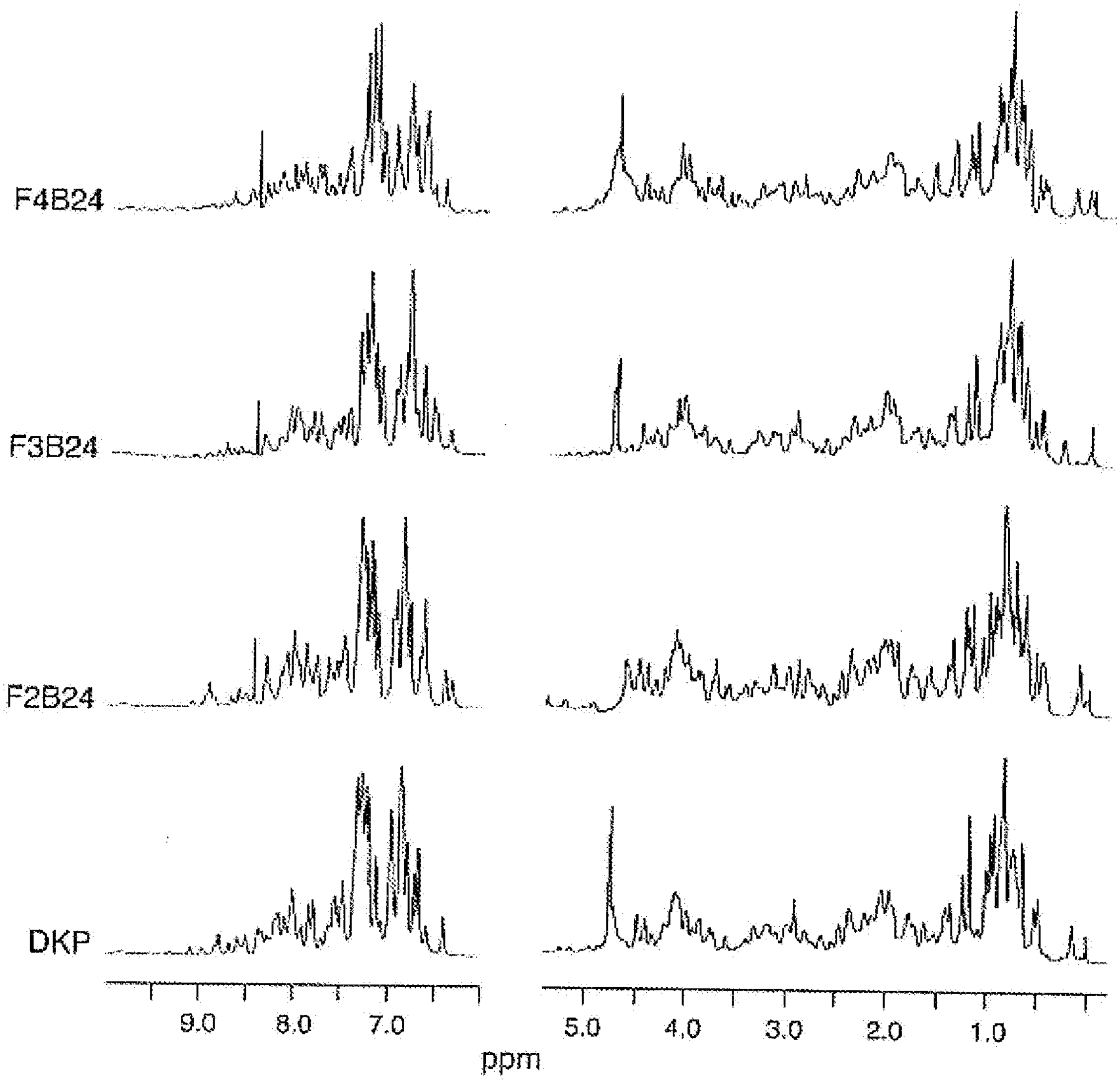


Fig. 8A

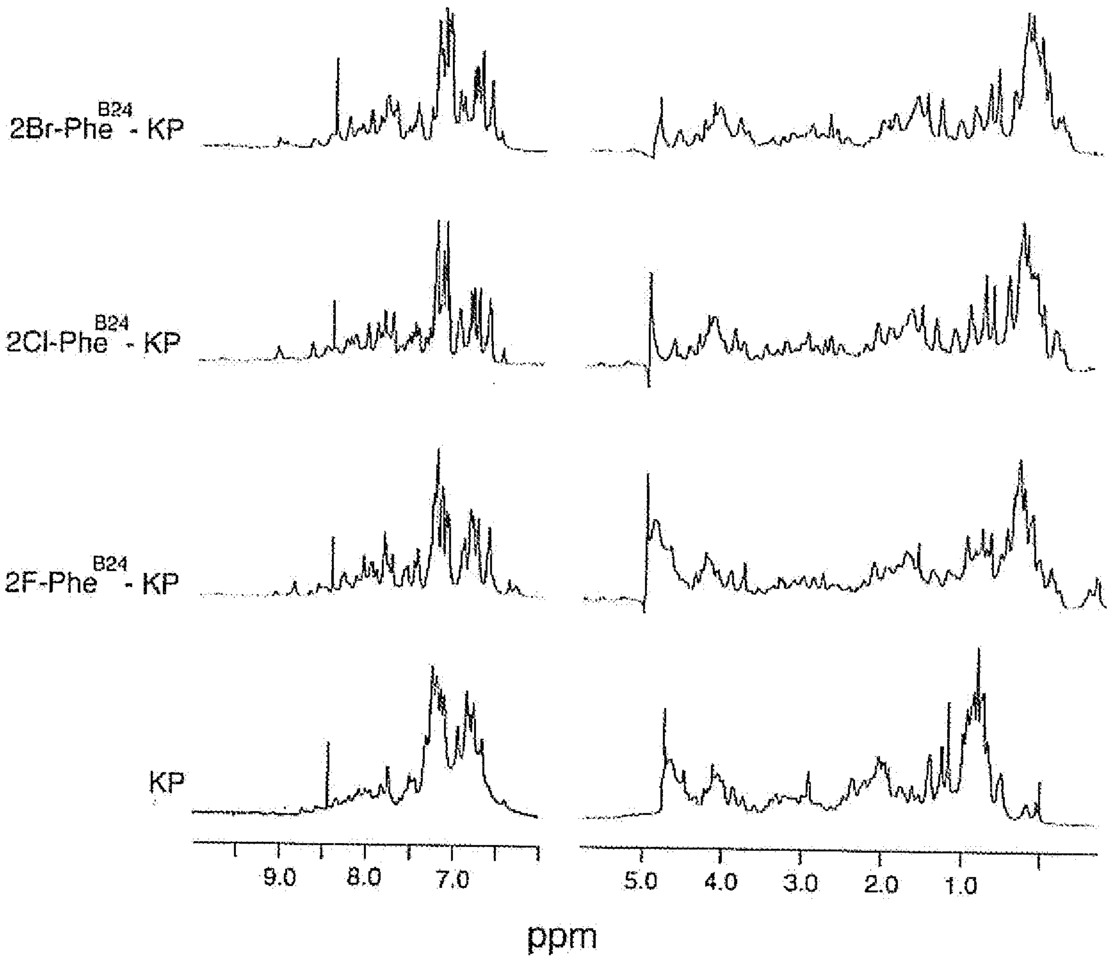
2Br-Phe$^{B24}$ - KP
2Cl-Phe$^{B24}$ - KP
2F-Phe$^{B24}$ - KP
KP
9.0
8.0
7.0
5.0
4.0
3.0
2.0
1.0
ppm

Fig. 8B

Europäisches Patentamt
European Patent Office
Office européen des brevets

# EUROPEAN SEARCH REPORT

Application Number
EP 15 18 3054

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HAIJUAN DU ET AL: "Insulin analogs with B24 or B25 phenylalanine replaced by biphenylalanine (pages 133 139)", ACTA BIOCHIMICA ET BIOPHYSICA SINICA, BLACKWELL PUBLISHING, INC., MALDEN, MA, US, vol. 40, no. 2, 1 February 2008 (2008-02-01), pages 133-139, XP008143460, ISSN: 1672-9145, DOI: 10.1111/J.1745-7270.2008.00379.X [retrieved on 2008-02-20] * the whole document * ----- | 1-15 | INV. C07K14/62 C12N15/17 A61K38/28 |
| A | WO 03/053339 A2 (LILLY CO ELI [US]; BEALS JOHN MICHAEL [US]; DEFELIPPIS MICHAEL ROSARIO) 3 July 2003 (2003-07-03) * the whole document * ----- | 1-15 | |
| A | ZAKOVA L ET AL: "Shortened insulin analogues: Marked changes in biological activity resulting from replacement of TyrB26 and N-methylation of peptide bonds in the C-terminus of the B-chain", BIOCHEMISTRY, AMERICAN CHEMICAL SOCIETY, US, vol. 43, no. 8, 2 March 2004 (2004-03-02), pages 2323-2331, XP002313978, ISSN: 0006-2960, DOI: 10.1021/BI036001W * the whole document * ----- | 1-15 | TECHNICAL FIELDS SEARCHED (IPC) C07K |
| | -/-- | | |

The present search report has been drawn up for all claims

1

| Place of search | Date of completion of the search | Examiner |
|---|---|---|
| Munich | 18 December 2015 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 18 3054

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MIRMIRA R G ET AL: "ROLE OF THE PHENYLALANINE B24 SIDE CHAIN IN DIRECTING INSULIN INTERACTION WITH ITS RECEPTOR", JOURNAL OF BIOLOGICAL CHEMISTRY, THE AMERICAN SOCIETY OF BIOLOGICAL CHEMISTS, INC, US, vol. 264, no. 11, 15 April 1989 (1989-04-15), pages 6349-6354, XP000007004, ISSN: 0021-9258 * the whole document * ----- | 1-15 | |
| Y | RAGHAVENDRA G. MIRMIRA ET AL: "Disposition of the phenylalanine B25 side chain during insulin-receptor and insulin-insulin interactions", BIOCHEMISTRY, vol. 30, no. 33, 20 August 1991 (1991-08-20), pages 8222-8229, XP055014070, ISSN: 0006-2960, DOI: 10.1021/bi00247a019 * the whole document * * page 8224 - page 8225; tables I,II * ----- | 1-15 | TECHNICAL FIELDS SEARCHED (IPC) |
| A | MIRMIRA R G ET AL: "IMPORTANCE OF THE CHARACTER AND CONFIGURATION OF RESIDUES B24 B25 AND B26 IN INSULIN-RECEPTOR INTERACTIONS", JOURNAL OF BIOLOGICAL CHEMISTRY, THE AMERICAN SOCIETY OF BIOLOGICAL CHEMISTS, INC, US, vol. 266, no. 3, 25 January 1991 (1991-01-25), pages 1428-1436, XP002313977, ISSN: 0021-9258 * the whole document * * page 1430 - page 1432; figure 2; tables I,II * ----- -/-- | 1-15 | |

The present search report has been drawn up for all claims

1

| Place of search | Date of completion of the search | Examiner |
|---|---|---|
| Munich | 18 December 2015 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 18 3054

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DUCKWORTH ET AL: "Degradation products of insulin generated by hepatocytes and by insulin protease.", JOURNAL OF BIOLOGICAL CHEMISTRY, vol. 263, no. 4, 5 February 1988 (1988-02-05), pages 1826-1833, XP055016909, ISSN: 0021-9258 * the whole document * ----- | 1-15 | |
| A | TERESA STELMASZYNSKA ET AL: "N-(2-Oxoacyl)amino Acids and Nitriles as Final Products of Dipeptide Chlorination Mediated by the Myeloperoxidase/H2O2/Cl- System", EUROPEAN JOURNAL OF BIOCHEMISTRY, vol. 92, no. 1, 1 December 1978 (1978-12-01), pages 301-308, XP055016913, ISSN: 0014-2956, DOI: 10.1111/j.1432-1033.1978.tb12748.x * the whole document * ----- | 1-15 | TECHNICAL FIELDS SEARCHED (IPC) |
| Y | G. CORNILESCU ET AL: "Solution structure of a small protein containing a fluorinated side chain in the core", PROTEIN SCIENCE, vol. 16, no. 1, 22 November 2006 (2006-11-22), pages 14-19, XP55234884, US ISSN: 0961-8368, DOI: 10.1110/ps.062557707 * the whole document * ----- | 1-15 | |

The present search report has been drawn up for all claims

1

| Place of search | Date of completion of the search | Examiner |
|---|---|---|
| Munich | 18 December 2015 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 18 3054

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2015

| Patent document cited in search report | Publication date | Patent family member(s) | Publication date |
|---|---|---|---|
| WO 03053339 A2 | 03-07-2003 | AU 2002346490 A1 | 09-07-2003 |
| | | BR 0215029 A | 20-12-2005 |
| | | CA 2468100 A1 | 03-07-2003 |
| | | CO 5590884 A2 | 30-12-2005 |
| | | CZ 20040710 A3 | 16-02-2005 |
| | | EP 1545460 A2 | 29-06-2005 |
| | | HR P20040551 A2 | 31-10-2004 |
| | | JP 2005519041 A | 30-06-2005 |
| | | KR 20040070237 A | 06-08-2004 |
| | | MX PA04006084 A | 31-03-2005 |
| | | SK 2432004 A3 | 01-04-2005 |
| | | US 2005014679 A1 | 20-01-2005 |
| | | WO 03053339 A2 | 03-07-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61085212 A
- US 5149777 A
- US 5474978 A
- US 0700320 W
- US 160187 A
- US 60828153 B
- US 07080467 W


### Non-patent literature cited in the description


- **MIRMIRA, R.G. ; TAGER, H.S.** *J. Biol. Chem.,* 1989, vol. 264, 6349-6354
- **MERRIFIELD et al.** *Biochemistry,* 1982, vol. 21, 5020-5031
- **WEISS et al.** *Biochemistry,* vol. 39, 15429-15440
- **SOSNICK et al.** *Methods Enzymol,* vol. 317, 393-409
- *J. Biol. Chem,* 2005, vol. 280, 20932-20936
- **WANG.** *FEBS Lett.,* 1995, vol. 360, 111-114
- **FURTER.** *Protein Sci.,* 1998, vol. 7, 419-426
- **XIE et al.** *Methods,* 2005, vol. 36, 227-238
- **FURTER, R.** Expansion of the genetic code: Site-directed p-fluoro-phenylalanine incorporation in Escherichia coli. *Protein Sci.,* 1998, vol. 7, 419-426
- **MERRIFIELD, R.B. ; VIZIOLI, L.D. ; BOMAN, H.G.** Synthesis of the antibacterial peptide cecropin A (1-33). *Biochemistry,* 1982, vol. 21, 5020-5031
- **MIRMIRA, R.G. ; TAGER, H.S.** Role of the phenylalanine B24 side chain in directing insulin interaction with its receptor: Importance of main chain conformation. *J. Biol. Chem,* 1989, vol. 264, 6349-6354
- **SOSNICK, T.R. ; FANG, X. ; SHELTON, V.M.** Application of circular dichroism to study RNA folding transitions. *Methods Enzymol,* 2005, vol. 317, 393-409
- **WANG, Z.X.** An exact mathematical expression for describing competitive biding of two different ligands to a protein molecule. *FEBS Lett,* 1995, vol. 360, 111-114
- **WEISS, M.A. ; HUA, Q.X. ; JIA, W. ; CHU, Y.C. ; WANG, R.Y. ; KATSOYANNIS, P.G.** Hierarchiacal protein ''un-design'': insulin's intrachain disulfide bridge tethers a recognition α-helix. *Biochemistry,* 2000, vol. 39, 15429-15440
- **WHITTAKER, J. ; WHITTAKER, L.** Characterization of the functional insulin binding epitopes of the full length insulin receptor. *J. Biol. Chem,* 2005, vol. 280, 20932-20936
- **XIE, J. ; SCHULTZ, P.G.** An expanding genetic code. *Methods,* 2005, vol. 36, 227-238